# EUROPEAN PATENT APPLICATION

(11) **EP 2 109 054 A1**
(43) Date of publication of application: **14.10.2009**
(21) Application number: 08154264.9
(22) Date of filing: 09.04.2008
(51) Int. Cl.: G06F 19/00

(54) **Methods for identifying biologically active peptides and predicting their function**

(71) Applicant: Biotempt B.V., 7958 NZ Koekange (NL)
(72) Inventor: Khan, Nisar Ahmed, 3039 JL Rotterdam (NL); Benner, Robbert, 2992 SH Barendrecht (NL); Wensvoort, Gert, 7958 NZ Koekange (NL); Smychliaev, Iaroslav Vladimirovitch, 3162 RA Rhoon (NL)
(74) Representative: Hatzmann, Martin

(57) **Abstract**

The invention relates generally to biotechnology, and more specifically to in *silico* methods of identifying lead molecules that have an increased probability of becoming an approved medicament, and business methods of identifying molecules such that they have an increased probability of becoming an approved medicament. Provided is a method for identifying a biologically active peptide consisting of two to seven amino acid residues, comprising the steps of providing a database comprising a plurality of naturally occurring polypeptide sequences; defining at least one peptide motif that satisfies certain defined criteria; and determining for the defined peptide motif its frequency of occurrence among the polypeptide sequences in the database and correlating the frequency with the biological activity of the peptide.

## Description

The invention relates generally to biotechnology and, more specifically, to methods of identifying lead molecules that have an increased probability of becoming an approved medicament, and business methods of identifying molecules such that they have an increased probability of becoming an approved medicament. Provided also are computer programs, data and databases, computer readable media, computer systems, and/or apparatus that use, compare or generate data relating to biologically active peptides. The invention can be used, interalia, for research, diagnostic and/or therapeutic products, methods and devices.

Drug discovery is typically a process that takes significant time and money. It may be broadly arranged according to disease target identification, target validation, identification of "hits" and "leads," lead optimization, and pre-clinical and clinical evaluation, with each area itself providing a vast discipline. Typically, the therapeutic profling process of characterizing the physical molecular properties of the molecules being pursued as potential drug candidates, such as absorption, distribution, metabolism, excretion (collectively referenced as "ADME"), and toxicity (T), consumes a majority of the time and expense associated with drug development. It has been estimated that the clinical failure of about 50% of the Investigational New Drug (IND) filings result from inadequate ADME and toxicity attributes.

Furthermore, the success rate in drug development has been estimated at approximately one marketed drug for every 6000 compounds that are synthesized and tested *(*DATA BOOK, 1999, Japan Pharmaceutical Manufacturers Association). It is no surprise that the pharmaceutical industry is searching for any means possible to minimize this attrition.

Mathematical models, sometimes referred to as in *silico* screens, have been the major focus of current efforts to predict ADME and/or toxicity attributes (ADME/T) solely from molecular structure.

Likewise, there is a similar problem associated with the identification of an appropriate drug target. The identification of appropriate drug targets is frequently complicated by the inability to predict alternative downstream events or to identify alternative sites of action. Until lead compounds can be selected or created based upon known properties desired in the eventual drug, undesirable side effects will remain a persistent problem.

The current invention relates to understanding and/or predicting the body's innate way of responding to small molecules and builds on insights reported in PCT International Publications WO99/59617 and WO01/72831 and PCT International Application PCT/NL02/00639, the contents of the entirety of all of which are incorporated herein by this reference. These applications describe small gene-regulatory peptides that are present in pregnant women and are derived from proteolytic breakdown of placental gonadotropins, such as hCG. These breakdown products are often only about three to six amino acids long and were shown to have unsurpassed immunological activity that is exerted by regulating expression of genes encoding inflammatory mediators such as cytokines. Surprisingly, it was found that breakdown of hCG provides a cascade of peptides that helps maintain a pregnant woman's immunological homeostasis. These peptides balance the immune system to assure that the mother stays immunologically sound while her fetus does not get prematurely rejected during pregnancy, but instead is safely carried until its time of birth. Other peptides known in the art have the antigenic binding activity of human chorionic gonadotropin (hCG). *See, e.g.,* U.S. Patent 5,380,668 to Herron (Jan. 10, 1995), the contents of the entirety of which are incorporated by this reference. The oligopeptides disclosed therein are disclosed generally for use in diagnostic methods.

Other patents and patent applications to Gallo et al. (e.g., U.S. Patent 5,677,275 (corresponding to WO 96/04008 A1), U.S. Patent 5,877,148 (also corresponding to WO 96/04008 A1), WO 97/49721 A1, U. S. Patent 6,319,504 (corresponding to WO 97/49373), U.S. Patent Application 2003/0049273 A1 (also corresponding to WO 97/49373), U.S. Patent 5,968,513 (corresponding to WO 97/49418), U.S. Patent 5,997,871 (corresponding to WO 97/49432), U.S. Patent 6,620,416, U.S. Patent 6,596,688, WO 01/11048 A2, WO 01/10907 A2., and U.S. Patent 6,583,109) relate to various oligopeptides and their use in, among other things, "inhibiting HIV infection," "treating or preventing HIV infection," "treating or preventing cancer," "treating or preventing a condition characterized by loss of body cell mass," "treating or preventing a condition associated with pathological angiogenesis," "treating or preventing hematopoietic deficiency," *"ex* vivo gene therapy," "expanding blood cells in *vitro,"* and/or "providing blood cells to a subject."

As may be expected in a complex biological response to numerous possible effector molecules (e.g., small gene-regulatory peptides), the number of effector molecules and the data obtainable for each molecule requires considerable time, skill, and resourses to generate or identify hit and lead compounds. Hence, there is a need in the art for improved methods of identifying hits and generating lead compounds for therapeutic purposes.

Where it was generally thought that the smallest breakdown products of proteins have no specific biological function on their own, it now emerges that the body may utilize the normal process of proteolytic breakdown to generate important compounds, such as small gene-regulatory peptides. We previously described that specific short breakdown products of hCG (*i.e.,* short peptides, derivates, or functional analogues which can easily be synthesized and used as a pharmaceutical composition) exert a major regulatory activity on pro- or anti-inflammatory cytokine cascades that are governed by a family of crucial transcription factors, for example, the NF-κB family, which generally regulate the expression of genes involved in the body's immune response. We now show that this specific phenomenon observed previously for hCG can be expanded to a more general principle. Provided herein are selection criteria that can be used to identify biologically active peptides derivable from among a library of proteins, for example from human proteins, bacterial proteins, viral proteins and the like.

The invention provides the insight that certain peptide motifs have an increased chance of being biologically active, in particular if they occur relatively frequently among naturally occurring proteins. It is the inventor's hypothesis that there is a pool of endogenous proteins which serves as a supplier of biologically active peptides. In a specific embodiment, a method of the invention identifies a biologically active peptide derivable from proteins other than hCG or from a fragment thereof, in particular, other than MTRVLQGVLPALPQWC.

In an exemplary embodiment, the invention provides a method for identifying one or more biologically active peptides consisting of two to seven amino acids, comprising the steps of providing a database comprising a plurality of naturally occurring polypeptide sequences, defining at least one peptide motif that satisfies one or more specific criteria as disclosed herein below, and determining for the defined peptide motif the frequency of occurrence among the polypeptides in the database, wherein the frequency of occurrence is indicative of the peptide motif having biological activity. The higher the frequency of a given peptide motif among an organism's proteasome, the higher the chance that the peptide is biologically active. Optionally, the results and/or physical characteristics are stored in a database as descriptors in a record associated with a particular peptide, wherein the database, or a subset of the database may be searched, sorted, or analyzed according to one or more descriptors, such as the effect of the peptide on production of nitric oxide (NO) or the levels of a tumor suppressor, to generate at least one hit and/or lead compound.

According to the invention, a biologically active peptide satisfies at least one or more of the following criteria: AP, PA, A(P)ₙ A or P(A)ₙ P, wherein n is 0, 1, 2, 3, 4 or 5 and wherein A stands for an amino acid residue selected from a first defined subset of "A" amino acids and wherein P stands for an amino acid residue selected from a second defined subset of "P" amino acids, wherein the first and second subset are different from each other. Examplary A and P amino acids are provided herein below.

In certain embodiments, provided is a method for predicting the biological function of a peptide that is or can be identified as being biologically active according to the above identification method of the invention. The prediction method comprises the steps of analyzing the frequency of occurrence of the peptide amino acid sequence of the biologically active peptide sequence among a database comprising multiple polypeptides, each having at least one known biological function; selecting from the database at least one polypeptide comprising at least one copy of the biologically active peptide sequence and identifying at least one biological network or pathway wherein the selected polypeptide is involved. According to the invention, the defined peptide is predicted to modulate at least one identified biological pathway or network.

In another embodiments, provided is a method for predicting the biological function of a peptide that is or can be identified as being biologically active according to the above identification method of the invention. The prediction method comprises the steps of analyzing the frequency of occurrence of the peptide amino acid sequence of the biologically active peptide sequence among a database comprising polypeptides which have as specific genetic trait one or more non-synonymous single nucleotide polymorpisms (SNP's) at their DNA coding for the amino acid sequence of said polypeptide. In this way a set or sets of corresponding peptides are generated that correspond to polypeptide sequences that are coded by DNA wherein said non-synonymous single nucleotide polymorpisms are detected. Such sets of peptides generally comprise two (occasionally there or more) peptides that differ in one amino acid, very occasionally they may differ in two or more amino acids. With the ever expanding knowledge on non-synonymous SNP's, it is here described that more and more disease is associated with those genetic traits, and said disease is associated with a lack (or presence) of said specific peptides predicted by the DNA around the SNP in question.

Also provided is a computer program (and associated equipment well known to those of skill in the art) capable of performing at least part of the steps comprised in the method for identifying a biologically active peptide and/or at least part of the steps comprised in the method of predicting the function of a biologically active peptide. Also provided are data and databases, computer readable media, computer systems, and/or apparatus that use, compare or generate data relating to a method according to the invention.

The invention provides at least one algorithm for generating at least one set of peptide motifs. For example, a Motif Search algorithm is provided that collects, classifies, analyzes and arranges protein sequence data in a particular output format that is based on one or more input criteria provided by the user. As another example, a Query Proteins algorithm is provided that scans submitted protein sequences for motif patterns and filters the results according to specified criteria.

The invention also provides a method of conducting a drug discovery business comprising i) identifying one or more biologically active peptides using a peptide identification method according to the invention, e.g., in the form of software stored on a computer system, ii) screening the peptide for the presence of descriptors indicative of a desirable therapeutic profile (e.g., ADME/T profile), iii) optionally modifying the peptide to improve its therapeutic profile; and iv) licensing, to a third party, the rights for further drug development of the peptide.

The method of conducting a drug discovery business may furthermore comprise predicting the biological function of the one or more biologically active peptides using a method of the invention and, optionally, correlating the predicted function with a disease or pathological condition.

In conducting therapeutic profiling of the peptide compound identified in step i), or further analogs thereof, for efficacy and toxicity in animals to generate lead compounds; iii) testing one or more lead compounds for efficacy and/or safety in human subjects, and iv) formulating and marketing a pharmaceutical preparation including one or more compounds of step iii), having an acceptable therapeutic profile. Such a business method can be further extended by including an additional step of establishing a distribution system for distributing the pharmaceutical preparation for sale, and may optionally include establishing a sales group for marketing the pharmaceutical preparation. Preferably, identifying lead peptide compounds includes screening hits for traits indicative of a desirable ADME/T profile and selecting compounds having a higher probability of exhibiting a pharmaceutically desirable ADME/T profile.

### LEGENDS TO THE FIGURES

While the specification concludes with claims particularly pointing out and distinctly claiming that which is regarded as the invention, the advantages of this invention can be more readily ascertained from the following description of the invention when read in conjunction with the accompanying drawings in which:
FIG. 1 is a graph comparing the number of motifs to the number of occurrences in a single protein.
FIG. 2A is a graph illustrating some of the results that were obtained when peptide motif ATFV.
FIG. 2B is another graph illustrating some of the results that were obtained when peptide motif ATFV.
FIG. 2C is another graph illustrating some of the results that were obtained when peptide motif ATFV.
FIG. 3A is a bar graph showing diseases and disorders.
FIG. 3B is a bar graph illustrating molecular and cellular functions.
FIG. 3C is a bar graph showing physiological systems development and functions.
FIG. 3D is a bar graph showing metabolic pathways.
FIG. 3E is a graph showing signalling pathways.
FIG. 4 is a flow chart for determining a Peptide-I database.
FIG. 5 is another flow chart for determining a Peptide-I database.

### DETAILED DESCRIPTION OF THE INVENTION

As used herein and in the appended claims, the singular forms *"a," "an,"* and *"the"* include plural reference unless the context clearly dictates otherwise. For example, reference to "a peptide" includes a plurality of such peptides and equivalents thereof known to those skilled in the art.

As used herein, *"record"* means a database entry for a particular compound, operably linking descriptors associated with the particular compound.

As used herein, *"descriptor"* means a metric used to describe a structure or certain molecular or functional attribute of a compound. Examples of descriptors and descriptor coding, include, but are not limited to: Hammin, Euclidean, Tversky, Tanimoto, Ghose and Crippen, and/or BCUT indices, molecular mass, calculated lipophilicity (e.g., LogP and LogD), rotatable bonds, polar surface area, molecular flexibility, SMILES, Bit Strings, pKa, hydrogen bond donors, hydrogen bond acceptors, total hydrogen bond count, individual amino acids by position in a polypeoptide, biomarker expression or activity, geometry-based descriptors, proteolytic cleavage sites, acid/base properties, conformational constraint, molecular topology, AUC (area under the drug plasma concentration-time curve), Cₘₐₓ (maximum drug concentration in plasma), adsorption properties, distribution properties, metabolism rates, excretion rates, toxicity, cardiotoxicity, nephrotoxicity, neurotoxicity, hepatotoxicity, electronegativity, polarity, solubility, membrane permeability, ability to cross the blood-brain barrier, mutagenicity, bioavailability, plasma protein binding, immunogenicity, drug interactions, etc.

As used herein, a *"purified* or *isolated"* peptide is a peptide that has been purified from a natural or biotechnological source.

As used herein *"Peptide," "Polypeptide"* and *"Protein"* include polymers of two, preferably three, or more amino acids, preferably the peptides are "virtual" (or *in silico)* breakdown products of a larger native protein. No distinction, based on length, is intended between a peptide, a polypeptide or a protein.

As used herein, a *"functional analogue"* or *"derivative"* of a peptide includes variations made with regard to a reference peptide, wherein the functional analoque or derivative retains an identifiable relationship to the reference peptide and retain the desired function of the reference peptide; functional analogues and derivatives include, but are not limited to, compounds having the same or equivalent sidechains as the reference peptide arranged sequentially in the same order as the reference peptide, but for example, joined together by non-peptide bonds (e.g., by isosteric linkages such as the keto isostere, hydroxy isostere, diketo isostere, or the keto-difluoromethylene isostere), non-naturally occurring amino acids or polyamides, surrogate peptide bonds (*see, e.g.,* U.S. Patent 6,689,753 to Soto-Jara, including, but are not limited to, CH₂, CH₂CH₂, CH=CH, C≡C, CH₂NH, COCH₂, CH₂S, CH₂SO₂, and NHCO replacement of amid bonds), amidation of one or more corbon substitution of an L-amino acid residue with a D-amino acid residue, amino acid substitutions (e.g., variations made by pepscan, ala-scanning, replacement net analysis, methods disclosed in U.S. Patent Application number 10/456,375 and/or conservative substitutions relative to the reference sequence), and/or conjugated to a sugar, lipid, another polypeptide, nucleic acid and/or PNA. A functional analogue or derivative may be considered a peptide for the purposes of screening, identification of activity, inclusion in a database, production of a pharmaceutical and used in the method of identifying hit and lead compounds.

Conservative amino acid substitutions are known in the art and generally constitute substitution of one amino acid residue with another residue having generally similar properties (size, hydrophobicity, or charge).

Further included within peptides, derivatives or functional analogues modifications, including, but not limited to, glycosylation, PEGylation, PEG alkylation, alkylation, acteylation, amidation, glycosyl-phophatdylinositalization, farnesylation, ADP-ribosylation, sulfation, lipid attachment, hydroxylation, and phosphorylation.

As used herein, *"compound"* and/or *"peptide"* includes an acceptable salt or ester of the compound or peptide. As understood in the art, an "acceptable salt or ester" refers to a salt or ester that retains the desired activity of the peptide or compound, and preferably does not detrimentally affect a subject, for example, a human subject. Examples of such salts are acid addition salts formed with inorganic acids, for example, hydrochloric acid, hydrobromic acid, sulfuric acid, phosphoric acid, nitric acid, and the like. Salts may also be formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminum, copper, cobalt, nickel and the like or with an organic cation formed from N,N'-dibenzylethylenediamine or ethylenediamine, or combinations thereof (e.g., a zinc tannate salt).

As used herein, a *"hit"* means a compound having a descriptor value meeting or exceeding a threshold value. As used herein, a *"lead"* means a compound or pharmacore structure having at least one descriptor value meeting or exceeding a threshold value and having at least one descriptor value related to an element of its therapeutic profile meeting or exceeding a threshold value. Examples of descriptors, include, but are not limited to, molecular mass, calculated lipophilicity (e.g., LogP and LogD), rotatable bonds, polar surface area, molecular flexibility, SMILES, Bit Strings, pKa, hydrogen bond donors, hydrogen bond acceptors, total hydrogen bond count, biomarker expression and/or activity, proteolytic cleavage sites, acid/base properties, conformational constraint, adsorption, distribution, metabolism, excretion, toxicity etc.

The invention provides a method for identifying (in *silico)* a biologically active peptide consisting of two to seven amino acid residues, comprising the steps of:
providing a database comprising a plurality of naturally occurring polypeptide sequences;
defining at least one peptide motif that satisfies one or more of the following criteria: AP, PA, A(P)ₙ A or P(A)ₙ P;
   wherein n = 0-5 and wherein A and P stand for amino acid residues selected from, respectively, a first group of defined A amino acids and a second group of defined P amino acids;
determining for the defined peptide motif its frequency of occurrence among the polypeptide sequences in the database and correlating the frequency with the biological activity of the peptide, wherein a frequency of at least one is indicative of the peptide having a biological activity.

The database comprising a plurality of naturally occurring polypeptide sequences may comprise wildtype and/or mutated polypeptides. The database can be generated manually or automatically.

It can be a commercial or a publicly available polypeptide database. In one embodiment, the database is a database with polypeptides of one or more defined organism(s) or group(s) of organism(s). For example, a database comprises human, viral, plant and/ or bacterial polypeptide sequences.

Of particular interest for the invention is a database which also contains information about the (predicted) biological function of the polypeptides. An exemplary database of use when practicing the invention is the UniProtKB/Swiss-Prot Protein Knowledgebase. This is an annotated protein sequence database established in 1986. The UniProtKB/Swiss-Prot Protein Knowledgebase is a curated protein sequence database that provides a high level of annotation, a minimal level of redundancy and a high level of integration with other databases. Together with UniProtKB/TrEMBL, it constitutes the UniProt Knowledgebase, one component of the Universal Protein Resource (UniProt), a one-stop shop allowing easy access to all publicly available information about protein sequences. It is maintained collaboratively by the Swiss Institute for Bioinformatics (SIB) and the European Bioinformatics Institute (EBI). The current Swiss-Prot Release is version 50.0 as of 30-May-2006, and contains 222289 entries.

A biologically active peptide is defined by one of the following motifs: AP, PA, A(P)ₙ A or P(A)ₙ P, wherein n is 0, 1, 2, 3, 4 or 5. Thus, peptides of the invention include peptide dimers AA and PP, peptide trimers APA and PAP, tetramers APPA and PAAP, pentamers APPPA and PAAAP, hexamers APPPPA and PAAAAP and heptamers APPPPPA and PAAAAAP.

According to the invention, A and P residues are selected from distinct, predetermined subsets of amino acid. The subsets may partially overlap with respect to the amino acids, i.e., a certain amino acid residue may be present in the subset of A amino acids as well as in the subset of P amino acids.

A and P may stand for amino acid residues with different physico- and/or chemical parameters. Physico- and/or chemical parameters based on which A and P can be defined comprise molecular mass (Dalton), surface area (Å²), volume (Å³), pKa carboxylic acid, pKb amine, pKa side-chain, isolectric point, solubility, density (crystal density, g/ml), non-polar surface area (Å²), estimated hydrophobic effect for residue or side chain burial (kcal/ml) and any combination thereof.

In one embodiment, A and P differ with respect to their ratio between the total surface area and the non-polar surface area, which is indicative of their relative polarity. For example, A is selected from amino acids Leu (L), Trp (W), Phe (F), Ile (I), Val (V), Pro (P), Ala (A), whereas P is selected from amino acids Tyr (Y), His (H), Thr (T), Lys (K), Ser (S), Arg (R), Glu (E), Gln (Q), Cys (C), Asp (D), Asn (N) and Gly (G).

In an alternative embodiment, A is selected independently from the subset consisting of amino acid residues A, V, L, I, P, M, F and W, whereas P is selected from the subset G, S, T, C, N, Q, Y, D, E, R, K and H.

In yet another embodiment, A is selected from the group consisting of amino acids Leu (L), Trp (W), Phe (F), Ile (I), Val (V), Pro (P), Ala (A), Met (M), Gly (G) and Cys (C); whereas P is selected from the group consisting of amino acids Tyr (Y), His (H), Thr (T), Lys (K), Ser (S), Arg (R), Glu (E), Gln (Q), Cys (C), Asp (D), Asn (N), Pro (P), Ala (A), Val (V), Gly (G), Phe (F) and Trp (W).

In certain embodiments, A is selected from the group consisting of amino acids Leu (L), Trp (W), Phe (F), Ile (I), Val (V), Pro (P), Ala (A), Met (M), Gly (G), Gln (Q) and Cys (C); whereas P is selected from the group consisting of amino acids Tyr (Y), His (H), Thr (T), Lys (K), Ser (S), Arg (R), Glu (E), Gln (Q), Cys (C), Asp (D), Asn (N), Pro (P), Ala (A), Val (V), Gly (G), Phe (F) and Trp (W).

Within a given peptide motif, A and P amino acids can be the same or they can be different. For example, motif APPA can have the sequence Trp-Arg-Arg-Val, Trp-Glu-Arg-Phe or Phe-Glu-Arg-Phe.

In view of the therapeutic application of a biologically active peptide identified according to the invention it is preferred that the peptide motif is smaller than seven amino acids (aa) because such a peptide generally does not binding to the MHC receptors, thereby decreasing the risk of the development of autoimmunity initiated by an immune response against the biologically active peptide when administered as a therapeutic agent.

This size of smaller than seven amino acids ("aa") is also particularly preferred because it was determined (when comparing peptides derived from the human proteome with those derived from pathogen proteomes, in particular of viruses or bacteria (Burroughs et al., Immunogenetics, 2004, 56:311-320)) that with a peptide size of seven aa only 3% overlap between self or non-self is found. For peptides of six aa, that overlap in human self with pathogen non-self was determined to be 30%, for peptides of five aa, 90%, and for four aa long (and smaller) peptides, 100% overlap between the peptides present in the human proteome and the peptides present in the proteome of pathogens was determined. Based on these data, it is now herein recognized that when the self-non-self difference is not present, risk of adverse immune reactions, such as anaphylactic shock, is greatly diminished, which is a distinct advantage when non-medically trained persons administer any drug to themselves or to others.

From the viewpoint of preventing adverse reactions such as anaphylactic shock it is thus preferred that the peptide consists of two to six amino acids, more preferably consists of three to five amino acids, and most preferably consists of three or four amino acids. From the viewpoint of activity, based on a general insight that activity is broader with increasing peptide size, if only to withstand full proteolysis longer whereby metabolic fragments of three aa still have activity, it is herein preferred that the peptide consists of four amino acids with the motif APPA or PAAP.

A method of the invention comprises the step of determining the frequency of occurrence of the at least one peptide motif, e.g., APA, among a database comprising multiple sequences of known proteins. The frequency is an indicator of the likelihood that the peptide is biologically active. The higher the frequency of a given peptide motif among the naturally occurring proteins, the higher the chance that the motif will be of biological significance.

In a specific embodiment, a method of the invention comprises the step of determining the frequency of occurrence of the at least one peptide motif among at least one single polypeptide sequence. Again, the higher the number of occurrence of a specific motif in a single protein, the more likely it is that the motif will be generated *in vivo* to exert a biological effect. Thus, not only the amino acid sequence of a peptide motif is an indicator of its biological activity but also its relative frequency of occurrence in the proteome.

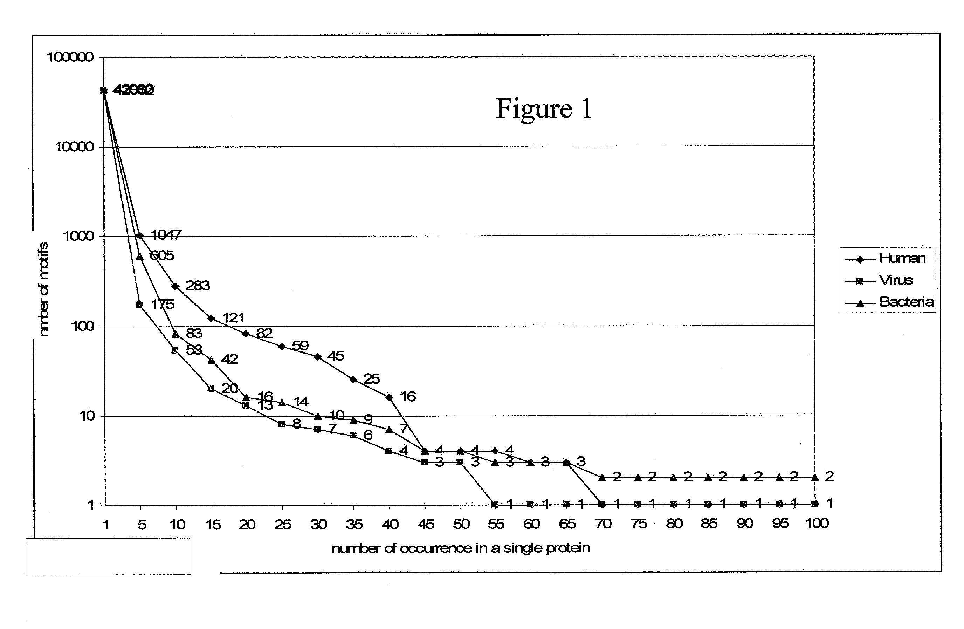

FIG. 1 shows as an example the results of determining the frequency of occurrence of tetrapeptide motif APPA among a database of human, viral or bacterial polypeptides. A was selected independently from amino acids L, W, F, I, V, P and A, while P was selected independently from amino acids Tyr (Y), His (H), Thr (T), Lys (K), Ser (S), Arg (R), Glu (E), Gln (Q), Cys (C), Asp (D), Asn (N) and Gly (G).

It was found that most of all possible tetrapeptide sequences that satisfy the above motif occur at least once among the polypeptides in the database. However, there is a steep drop in the number of motifs that are found more frequently among a single peptide. For instance, among the human protein database comprising over 14000 polypeptide entries, there are only around 1000 different APPA peptide motifs occur five times or more within a single polypeptide. A nearly four-fold further reduction is observed in the number of peptides which occur at least ten times. A similar trend is observed for the other organisms.

This indicates that the frequency of occurrence of a given peptide sequence among naturally occurring polypeptides is not just a matter of chance or coincidence. Rather, it suggests that polypeptides which occur in nature are "enriched" with only a limited number of different peptide motifs such that upon degradation of the polypeptide(s) a specific pool of breakdown products having biological activity is formed.

In one embodiment, a method of the invention for identifying a biologically active peptide comprises selecting a peptide motif, for instance a tetrapeptide motif, having a frequency of occurrence among a single polypeptide sequence of at least five, preferably at least ten. As can be derived from FIG. 1, less than 50 of all APPA motifs occur more than 30 times within a single human polypeptide and less than 16 tetrapeptides are represented more than 40-fold in one protein.

For example, peptide motifs LTSL, FVLS, NMWD, LCFL, MWDF, FSYA, FWVD and AFTV can be identified to be present in the naturally occurring polypeptide C-Reactive Protein (CRP) (e.g., human CRP), peptide motifs GLLG, TAPS, VCQV, CLWT, VHQL, GALH, LGTL, TLVQ, QLLG, YAIT, LCEL, GLIR, APSL, ITTL, QALG, HPPS, GVLC, LCPA, LFYA, NIMR, NLIN, LHPP, LTEL, SPIE, VGGI, QLLY, LNTI, LWTL, LYSP, YAMT, LHNL, TVLR, and LFYA are present in Beta-catenin (e.g., human CTNB); peptide motifs LSNI, YVFS, LYGV, YVVC, FIVR, NILD, TIMY, LESI, FLLT, VFSP, FILE, TFLK, FWID, MWEI, QLLE, PCFW, VHKL, LYGV, LESI, LSNI, YVFS, IYSL, and NILD are present in Bruton's tyrosine kinase (e.g., human BTK), and peptide motifs FKGA, FFGL, GIAQ, LGCL, YWIY, AWNA, ARGA, PFRF, APSP CLLS, GLPQ, TFWP, AYYL, FWPE, CLLG, FLWC, RIIG, WSDV, PIIK, GLPP, RALC, LNTF, LSHA, ATFW, PSPI, AHEF, WRTV, FVLK, VQYL, KFFG, FPFR, IYSA, and FDGI can be found in matrix metalloproteinase-2 (e.g., human MM02).

As will be understood, a relatively large peptide motif of the invention, like a peptide consisting of six or seven amino acids, has a smaller statistical chance to be present in a polypeptide as compared to a relatively small peptide motif, for example a di- or tripeptide motif. The correlation between a given number of occurrences of a peptide motif among a single polypeptide and the biological activity of the peptide motif may therefore depend on the peptide size. In other words, a frequency of occurrence of five or more may not be a discriminant for biological activity of a tripeptide whereas it is a strong indicator for a hexapeptide.

In one embodiment, a biologically active dipeptide is identified by determining which of the dipeptide sequences according to the AP, PA, AA or PP motif is present at least 30 times, preferably at least 50 times, more preferably at least 70 times among a single naturally occurring polypeptide sequence.

In certain embodiments, a biologically active tripeptide is identified by determining which of the tripeptide sequences according to the APA or PAP motif is present at least 20 times, preferably at least 30 times, more preferably at least 50 times among a single naturally occurring polypeptide sequence.

In yet another embodiment, a biologically active tripeptide is identified by determining which of the tetrapeptide sequences according to the APPA or PAAP motif is present at least ten times, preferably at least 20 times, more preferably at least 30 times among a single naturally occurring polypeptide sequence.

In yet another embodiment, a biologically active pentapeptide is identified by determining which of the pentapeptide sequences according to the APPPA or PAAAP motif is present at least five times, preferably at least ten times, more preferably at least 20 times among a single naturally occurring polypeptide sequence.

In a further embodiment, a biologically active hexapeptide is identified by determining which of the hexapeptide sequences according to the APPPPA or PAAAAP motif is present at least three times, preferably at least seven times, more preferably at least ten times among a single naturally occurring polypeptide sequence.

In a still further embodiment, a biologically active heptapeptide is identified by determining which of the heptapeptide sequences according to the APPPPPA or PAAAAAP motif is present at least two times, preferably at least five times, more preferably at least seven times among a single naturally occurring polypeptide sequence.

According to the invention, the term "biological activity" refers to any activity which regulates or influences, either in a transient or sustained fashion, at least one cellular process or pathway. The regulation or influence can be inhibitory (suppressive) or stimulatory. The cellular process or pathway can be a normal or a pathogenic pathway, for example a signal transduction pathway involved in inflammatory conditions or oncogenesis.

In one embodiment, the invention provides a method for identifying a peptide which exerts at least part of its biological activity in the nucleus of a cell. For instance, the peptide is involved in the regulation of the nuclear transcription machinery.

In certain embodiments, the invention provides a method for identifying a peptide which exerts at least part of its biological activity in the cytoplasm (also known as cytosol) of a cell. For example, it is involved in the regulation of translation machinery and/or in one or more cytosolic signal transduction pathway(s).

Of course, a peptide may also exert its biological activity in multiple different cellular compartments. In one embodiment, it shuttles between the cell nucleus and the cytoplasm.

The manner by which the peptide exerts its biological activity or biologicl activities can vary. It can be via a physical interaction with a cellular polypeptide or with a polynucleotide (DNA, RNA), or a combination thereof such as binding to a DNA-protein complex.

It was found that peptide motifs which satisfy the criterium A(P)ₙA wherein n= 1-5 are particularly good candidates for exerting a biological effect via a direct interaction with a polynucleotide. Accordingly, in one embodiment the invention provides a method for identifying a peptide consisting of two to seven amino acid residues having a nuclear biological activity, comprising the steps of (i) providing a database comprising a plurality of naturally occurring polypeptide sequences; (ii) defining at least one peptide motif that satisfies the criterium A(P)ₙ A, wherein n = 1-5 and wherein A and P stand for amino acid residues with different physico- and/or chemical parameters; (iii) determining for the defined peptide motif its frequency of occurrence among the polypeptide sequences in the database and correlating the frequency with the biological activity of the peptide, wherein a frequency of at least one is indicative of the peptide having a biological activity.

Table 1 shows exemplary tetrapeptides having nuclear activity. "A" amino acid residues were selected from LMAPVWIFCGQ, whereas "P" amino acid residues were selected from QTAPGRVCWFDHNSKYE. SwissProt database was used to identify the relative occurrence in human polypeptides. Indicated are those peptide motifs that occur with a frequency of at least one.

**Table 1: Tetrapeptides predicted to have nuclear activity**

| **Motif** | **Frequency** | **Motif** | **Frequency** | **Motif** | **Frequency** |
|---|---|---|---|---|---|
| GGGG | 14 | NFGG | 1 | MGAG | 1 |
| PPPP | 10 | GGLF | 1 | LQQQ | 1 |
| QQQQ | 4 | QKEQ | 1 | TLLS | 1 |
| GGYG | 3 | QALQ | 1 | LETL | 1 |
| PQAP | 2 | QEQL | 1 | HLAK | 1 |
| QAPG | 2 | LENL | 1 | LETI | 1 |
| QPPP | 2 | NLQD | 1 | PGGL | 1 |
| PRPP | 2 | IKEL | 1 | QLQA | 1 |
| APPP | 2 | LSEL | 1 | LEEL | 1 |
| APGV | 2 | AFGT | 1 | VKKL | 1 |
| PWQQ | 2 | QEQA | 1 | GNFG | 1 |
| PPAP | 2 | LEAL | 1 | ALQE | 1 |
| PVGP | 2 | GMGG | 1 | TPGH | 1 |
| FGGG | 2 | VHPP | 1 | MGPA | 1 |
| GPPP | 2 | VHPQ | 1 | GRGG | 1 |
| PAPG | 2 | SVLQ | 1 | TPGA | 1 |
| PPGV | 2 | LESL | 1 | ELLQ | 1 |
| GYGG | 2 | ELLK | 1 | PPPM | 1 |
| GFGG | 2 | LESQ | 1 | AEQL | 1 |
| AQVA | 2 | EALK | 1 | ELQS | 1 |
| AAAA | 2 | QLQS | 1 | PPPE | 1 |
| PPPG | 2 | GGNF | 1 | Total: 120 | 19 |
| GGSG | 2 | PVPR | 1 | | |
| LEDL | 1 | QLQD | 1 | | |
| TQLE | 1 | VERM | 1 | | |
| LQSV | 1 | HPPA | 1 | | |
| LKEV | 1 | IERM | 1 | | |
| RMGP | 1 | ATPG | 1 | | |
| PPPS | 1 | LERM | 1 | | |
| LKQQ | 1 | AGMG | 1 | | |
| QQLQ | 1 | VASL | 1 | | |
| TGFG | 1 | KLQS | 1 | | |
| HPQA | 1 | AQQL | 1 | | |
| LEQA | 1 | LEDA | 1 | | |
| SLLK | 1 | SLLE | 1 | | |
| QAQL | 1 | GFGT | 1 | | |
| TGLF | 1 | GVHP | 1 | | |
| LQEL | 1 | QSSL | 1 | | |
| ELAE | 1 | LKEL | 1 | | |
| EALE | 1 | GLFG | 1 | | |
| RMGA | 1 | RGFG | 1 | | |
| FGTA | 1 | SLQD | 1 | | |
| PGVH | 1 | GGGN | 1 | | |
| LTAQ | 1 | QLIE | 1 | | |
| PLPP | 1 | TLLE | 1 | | |
| LQDA | 1 | YGGG | 1 | | |
| PMPP | 1 | QTEQ | 1 | | |
| RMGS | 1 | TAQV | 1 | | |
| PPRP | 1 | YGGS | 1 | | |
| | | KFLE | 1 | | |

Furthermore, it was found that peptide motifs which satisfy the criterium P(A)ₙP wherein n= 1-5, are particularly good candidates for exerting a biological effect by binding to another protein, i.e., via a protein-protein interaction. Protein-protein interactions mainly occur in the cytosol, for example in processes such as translation and signal transduction. Accordingly, in one embodiment the invention provides a method for identifying a peptide consisting of two to seven amino acid residues having a cytosolic biological activity, comprising the steps of (i) providing a database comprising a plurality of naturally occurring polypeptide sequences; (ii) defining at least one peptide motif that satisfies the criterium P(A)ₙP, wherein n = 1-5 and wherein A and P stand for amino acid residues with different physico- and/or chemical parameters; (iii) determining for the defined peptide motif its frequency of occurrence among the polypeptide sequences in the database and correlating the frequency with the biological activity of the peptide, wherein a frequency of at least one is indicative of the peptide having a biological activity.

Table 2 shows exemplary tetrapeptides predicted to have an effect on the translation machinery. "A" amino acid residues were selected from LMAPVWIFCGQ, whereas "P" amino acid residues were selected from QTAPGRVCWFDHNSKYE. SwissProt database was used to identify the relative occurrence in human polypeptides. Only those motifs having a frequency of occurrence of two or more are shown.

**Table 2: Tetrapeptides predicted to have translational activity**

| **Motif** | **Frequency** | **Motif** | **Frequency** | **Motif** | **Frequency** | **Motif** | **Frequency** |
|---|---|---|---|---|---|---|---|
| AAAA | 134 | AGGA | 4 | PGGG | 2 | AGGG | 2 |
| PPPP | 86 | AQAQ | 4 | ACGK | 2 | PSPA | 2 |
| GGGG | 68 | AQAA | 4 | IVHQ | 2 | GPAQ | 2 |
| QQQQ | 66 | PYVC | 4 | PFAC | 2 | SPGP | 2 |
| GEKP | 52 | APAA | 4 | GECG | 2 | CGKV | 2 |
| ECGK | 45 | PPLP | 4 | NACG | 2 | VWFQ | 2 |
| IHTG | 38 | AAPP | 4 | IRHQ | 2 | PFVC | 2 |
| CGKA | 37 | PPAP | 4 | APPQ | 2 | QGPP | 2 |
| GKAF | 35 | DCGK | 4 | VQGQ | 2 | ASAA | 2 |
| PYEC | 22 | AAAS | 4 | QVQA | 2 | PGPP | 2 |
| KAFS | 17 | AFNQ | 4 | PPPL | 2 | PGPS | 2 |
| PYKC | 17 | AQQQ | 4 | ECGQ | 2 | QGQV | 2 |
| CNEC | 14 | PPGP | 4 | IHSG | 2 | QGQQ | 2 |
| GERP | 11 | PPGF | 4 | ASSP | 2 | PGPG | 2 |
| GKSF | 11 | GGTC | 3 | KAFR | 2 | PCQN | 2 |
| QPQP | 10 | VDEC | 3 | PPGG | 2 | IHAG | 2 |
| PAPA | 10 | APSP | 3 | KAFK | 2 | GKVF | 2 |
| APAP | 10 | LEDG | 3 | HAGE | 2 | PSSP | 2 |
| GAGG | 8 | QQQL | 3 | AAAP | 2 | QLQQ | 2 |
| CKEC | 8 | NGGT | 3 | AAAV | 2 | GPAA | 2 |
| QQQP | 8 | GKGF | 3 | PQQP | 2 | GFTG | 2 |
| CEEC | 8 | PSPG | 3 | AAAG | 2 | QQGP | 2 |
| KAFN | 7 | CGKG | 3 | CPEC | 2 | IDDC | 2 |
| GKTF | 6 | SAAA | 3 | QQPQ | 2 | GAQA | 2 |
| PQQQ | 6 | QQLQ | 3 | GGFG | 2 | QAQV | 2 |
| QQPP | 6 | KAFT | 3 | PAAP | 2 | GAQP | 2 |
| PQPQ | 6 | CGEC | 3 | PQPP | 2 | KPFK | 2 |
| CSEC | 6 | CQNG | 3 | GGGP | 2 | GGSG | 2 |
| GGAG | 6 | KIIH | 3 | ECGE | 2 | PKPW | 2 |
| AGAG | 6 | GPSP | 3 | GGGA | 2 | LPPP | 2 |
| GPGP | 6 | KGFS | 3 | GGPG | 2 | APGP | 2 |
| AFSQ | 6 | IDEC | 2 | GGPP | 2 | ECAS | 2 |
| CPPG | 6 | GRGF | 2 | PPQP | 2 | PPAQ | 2 |
| QPPP | 6 | GAGA | 2 | CAPG | 2 | GYTG | 2 |
| GPPG | 6 | SGGG | 2 | VQAQ | 2 | LYKC | 2 |
| VHTG | 5 | QNGG | 2 | PTPP | 2 | PYAC | 2 |
| IQHQ | 4 | GPRC | 2 | PAQP | 2 | ECGR | 2 |
| APPP | 4 | VFGG | 2 | QPQQ | 2 | AASA | 2 |
| GGGS | 4 | INEC | 2 | PAPA | 2 | LQQQ | 2 |
| GPGG | 4 | GFGG | 2 | PPPS | 2 | PGIP | 2 |
| PPQQ | 4 | CREC | 2 | PPPQ | 2 | GPPP | 2 |
| AVAA | 4 | PFKC | 2 | PAPP | 2 | CGRG | 2 |
| PPPA | 4 | AQVQ | 2 | CLPG | 2 | AGPG | 2 |
| AAGA | 4 | VGAG | 2 | PASP | 2 | AAQA | 2 |
| QAQA | 4 | TPAP | 2 | QQVQ | 2 | PQGP | 2 |
| QCGK | 4 | LPGF | 2 | GAAG | 2 | FSKV | 2 |
| | | | | | | AQPP | 2 |
| | | | | | | VQQV | 2 |
| | | | | | | AFIQ | 2 |
| | | | | | | CLCP | 2 |
| | | | | | | QQGQ | 2 |
| | | | | | | PVAP | 2 |
| | | | | | | PAAA | 2 |
| | | | | | | TALH | 2 |
| | | | | | | PECG | 2 |
| | | | | | | AAVA | 2 |
| | | | | | | GEKL | 2 |
| | | | | | | QAPP | 2 |
| | | | | | | PAAS | 2 |
| | | | | | | KALE | 2 |
| | | | | | | QGSV | 2 |
| | | | | | | QAAA | 2 |
| | | | | | | ACPP | 2 |

Table 3 shows exemplary tetrapeptides predicted to have an effect of the transcription machinery. "A" amino acid residues were selected from LMAPVWIFCGQ, whereas "P" amino acid residues were selected from QTAPGRVCWFDHNSKYE. SwissProt database was used to identify the relative occurrence in human polypeptides.

**Table 3: Tetrapeptides predicted to have transcriptional activity**

| **Motif** | **Frequency** | **Motif** | **Frequency** | **Motif** | **Frequency** |
|---|---|---|---|---|---|
| AAAA | 8 | ASQG | 1 | VSFV | 1 |
| PPPP | 4 | NVWR | 1 | SFVN | 1 |
| PPPQ | 2 | FPAY | 1 | KIVG | 1 |
| TAAT | 2 | SQIH | 1 | LPEG | 1 |
| FVGG | 2 | TGVS | 1 | GPWR | 1 |
| VFVG | 2 | SPFQ | 1 | KLGP | 1 |
| PFQV | 2 | GRYG | 1 | GSCF | 1 |
| PPQF | 2 | RGLD | 1 | TVFV | 1 |
| AFPA | 2 | WRNP | 1 | GWVL | 1 |
| GGPG | 2 | EIGS | 1 | NPPP | 1 |
| QAFP | 2 | PNPV | 1 | AASQ | 1 |
| AAAP | 2 | DVQK | 1 | IGSC | 1 |
| GPGG | 2 | SQGW | 1 | QNVW | 1 |
| PAAA | 2 | YLQP | 1 | FQNV | 1 |
| QGWV | 2 | KLCA | 1 | PNTV | 1 |
| PGPG | 2 | LGPA | 1 | PNSP | 1 |
| PQFQ | 2 | GPAI | 1 | LQPQ | 1 |
| ATTA | 1 | WNP | 1 | PQIT | 1 |
| GKKL | 1 | HVQP | 1 | YQAF | 1 |
| ALAS | 1 | QFQN | 1 | FVSF | 1 |
| VNDV | 1 | GSQI | 1 | PRRG | 1 |
| VSKG | 1 | SAAN | 1 | GGID | 1 |
| IRKQ | 1 | SAAA | 1 | VDVQ | 1 |
| QLPV | 1 | CFGR | 1 | WRRG | 1 |
| PETP | 1 | RPLV | 1 | Total: 112 | 12 |
| NPVT | 1 | WVLP | 1 | | |
| RGPR | 1 | SCFG | 1 | | |
| QPRP | 1 | PLVV | 1 | | |
| VKEV | 1 | VQPR | 1 | | |
| PRPL | 1 | PPGE | 1 | | |
| PAAE | 1 | VGGI | 1 | | |
| QPQI | 1 | FQVT | 1 | | |
| PAYP | 1 | PAIR | 1 | | |
| VGSQ | 1 | VNPP | 1 | | |
| YQLP | 1 | ASTQ | 1 | | |
| GFVS | 1 | GYGF | 1 | | |
| RGMD | 1 | GYQF | 1 | | |
| VQKI | 1 | QHVQ | 1 | | |
| KIVP | 1 | YGFV | 1 | | |
| AANP | 1 | GYQL | 1 | | |
| VLPE | 1 | AAAS | 1 | | |
| AAVT | 1 | ARHV | 1 | | |
| PVTQ | 1 | KIIT | 1 | | |
| PTPP | 1 | VTTG | 1 | | |

As mentioned above, it is herein disclosed that certain peptide motifs can be identified as being biologically active based on their relative frequency of occurrence in natural proteins. The present inventors identified as a further criterium for a peptide motif to be biologically active the likelihood that the peptide is generated in *vivo,* for example during intracellular protein turnover. According to the invention, a peptide motif has an increased chance of being biologically active if its complete sequence is present at least once, preferably at least twice, more preferably at least five times, within a fragment of a polypeptide that is or can be generated *in vivo.* In contrast, peptide motifs that are likely to be degraded, for instance because they contain an enzymatic cleavage site, are less likely to be biologically active.

The invention therefore relates to an identification method as described herein above with the additional step or process of determining the likelihood that the peptide is generated *in vivo,* or, in other words, the likelihood that the peptide motif remains intact upon degradation of the polypeptide comprising the motif sequence. The process comprises the steps of: a) selecting from the database at least one polypeptide sequence comprising at least one copy of at least one defined peptide motif; b) determining in at least one selected polypeptide the presence of one or more polypeptide fragments having an increased likelihood of being generated *in vivo;* and c) selecting at least one defined peptide motif whose amino acid sequence is present in at least one of the polypeptide fragments. According to the invention, an increased likelihood is a further positive indicator of the peptide having a biological activity.

Step b) of determining what fragment(s) a polypeptide have an increased chance of being formed within a cell may be based on one or more known biological phenomena related to selective or aselective protein breakdown. For instance, it comprises determining in *silico* the presence of a polypeptide fragment which is flanked by one (*e.g.,* C or N-terminal fragment) or more predicted cleavage sites.

The cleavage sites can be an enzymatic cleavage site, preferably a cleavage site recognized by an enzyme selected from the group consisting of the proteasome, immunoproteasome, Arg-C proteinase, Asp-N endopeptidase, caspase, Chymotrypsin (high specificity (C-term to [FYW], not before P) or low specificity (C-term to [FYWML], not before P), Clostripain (Clostridiopeptidase B, Enterokinase, Factor Xa, Glutamyl endopeptidase, GranzymeB, LysC, Pepsin (pH1.3), Pepsin (pH>2), Proline-endopeptidase, Proteinase K, Staphylococcal peptidase I, Thermolysin, Thrombin and Trypsin.

Enzymatic cleavage sites within a polypeptide can be readily identified in *silico* using publicly available software. For example, "Cutter" is a program that allows to generate peptide fragments by the enzymatic or chemical cleavage of a protein sequence entered by the user, and that computes the theoretical masses of the generated peptides. It can be accessed at http://delphi:phys.univ-tours.fr/Prolysis/cutter.html.

In a further embodiment, the cleavage site is a cleavage site for a chemical agent, for example BNPS-Skatole, CNBr, Formic acid, Hydroxylamine, Iodosobenzoic acid and/or NTCB (2-nitro-5-thiocyanobenzoic acid).

Once the "virtual" proteolytic fragment(s) of a polypeptide comprising ta least one intact copy of the defined peptide motif(s) have been identified, the fragments are preferably subjected to an analysis to determe whether the fragment(s) are likely to be generated in the antigen-processing pathway. If this is the case, it is another positive indicator of the peptide motif having biological activity.

Antigen processing and presentation are processes that occur within a cell that result in fragmentation (proteolysis) of proteins, association of the fragments with MHC molecules, and expression of the peptide-MHC molecules at the cell surface where they can be recognized by the T cell receptor on a T cell. However, the path leading to the association of protein fragments with MHC molecules differs for class I and class II MHC. MHC class I molecules present degradation products derived from intracellular (endogenous) proteins in the cytosol. MHC class II molecules present fragments derived from extracellular (exogenous) proteins that are located in an intracellular compartment.

In one embodiment, a method of the invention comprises the use of a computational prediction method for MHC class I and/or class II binding peptides. The presence of one or more copies of a specific peptide motif in a polypeptide fragment that is predicted to bind to MHC class I and/or class II is a (further) positive indicator of the peptide motif having biological activity.

In one aspect, a method for identifying a peptide of two to seven amino acids having biological activity involves determining in *silico* the presence of the motif in a polypeptide region that is a predicted binder to at least one class I MHC allele. For example, the binding regions of a polypeptide comprising one or more copies of the defined peptide sequence (e.g., APPA or PAAAP) to at least one HLA allele is determined.

Preferably, the polypeptide region binding to at least one HLA allele selected from from the group consisting of HLA-alleles HLA-A*1101, HLA-A2.1, HLA-A*3302, HLA-B14, HLA-B*3701, HLA-B40, HLA-B*5103, HLA-B*51, HLA-B62, HLA-Cw*0301, H2-Db, H2-Kd, HLA-A2, HLA-A24, HLA-A68.1, HLA-B*2702, HLA-B*3801, HLA-B*4403, HLA-B*5201, HLA-B*5801, HLA-B7, HLA-Cw*0401, H2-Db, H2-Kk, HLA-A*0201, HLA-A3, HLA-A20 cattle, HLA-B*2705, HLA-B*3901,HLA-B*5101, HLA-B*5301, HLA-B60, HLA-B*0702, HLA-Cw*0602, H2-Ld, HLA-A*0205, HLA-A*3101, HLA-B*3501, HLA-B*3902, HLA-B*5102, HLA-B*5401, HLA-B61, HLA-B8,HLA-Cw*0702 and H2-Dd,H2-Kb.

In certain embodiments, a method for identifying a biologically active peptide involves selecting from a database at least one polypeptide sequence comprising at least one copy of at least one defined peptide motif; determining in at least one selected polypeptide the presence of one or more polypeptide fragments having an increased likelihood of binding to at least one class II MHC allele, and selecting at least one defined peptide motif whose amino acid sequence is present in at least one of the polypeptide fragments.

The polypeptide fragment(s) are for example predicted binders to at least one class II MHC allele selected from the group consisting of HLA-alleles HLA-DR1, HLA-DRB1*0101, HLA-DRBI*0102, HLA-DR3 HLA-DRB1*0301, HLA-DRB1*0305, HLA-DRB1*0306, HLA-DRB1*0307, HLA-DRB1*0308, HLA-DRB1*0309, HLA-DRB1*0311, HLA-DR4, HLA-DRB1*0401, HLA-DRB1*0402, HLA-DRB1*0404, HLA-DRB1*0405, HLA-DRB1*0408, HLA-DRB1*0410, HLA-DRB1*0423, HLA-DRB1*0426, HLA-DR7, HLA-DRB1*0701, HLA-DRB1*0703, HLA-DR8, HLA-DRB1*0801, HLA-DRB1*0802, HLA-DRB1*0804, HLA-DRB1*0806, HLA-DRB1*0813, HLA-DRB1*0817, HLA-DR11, HLA-DRB1*1101, HLA-DRB1*1102, HLA-DRB1*1104, HLA-DRB1*1106, HLA-DRB1*1107, HLA-DRB1*1114, HLA-DRB1*1120, HLA-DRB1*1121, HLA-DRB1*1128, HLA-DR13, HLA-DRB1*1301, HLA-DRB1*1302, HLA-DRB1*1304, HLA-DRB1*1305, HLA-DRB1*1307, HLA-DRB1*1311, HLA-DRB1*1321, HLA-DRB1*1322, HLA-DRB1*1323, HLA-DRB1*1327, HLA-DRB1*1328, HLA-DR2, HLA-DRB1*1501, HLA-DRB1*1502, HLA-DRB1*1506, HLA₋DRB5*0101 and HLA-DRB5*0105.

Various computational methods for predicting the MHC binding capacity of a given peptide sequence are known in the art. Prediction of MHC-peptides can be divided into two groups: sequence based and structure based methods. Allele specific sequence motifs can be identified by studying the frequencies of amino acids in different positions of identified MHC-peptides. The peptides that bind to HLA-A*0201 are often nine amino acids long (nonamers), and frequently have two anchor residues, a lysine in position 2 and a Valine in position 9 (Rotzschke et al., European Journal of Immunology 1992, 22:2453-2456). This type of sequence patterns has been used as a simple prediction method (Rammensee et al., Immunogenetics 1995, 41:962-965). Besides the anchor residues, there are also weaker preferences for specific amino acids in other positions. One method to include this information is to use a profile, where a score is given for each type of amino acid in each position. The scores can be calculated from observed amino-acid frequencies in each position or be set manually. The sum of the scores for a given peptide is then used to make predictions.

One frequently used profile based prediction method is SYFPEITHI, which is freely available as a web service at http://www.syfpeithi.de/. SYFPEITHI prediction can be done for different MHC class I and II types.

Another profile based MHC-peptide predictor is HLA_BIND at http://bimas.dcrt.nih.gov/molbio/hla_bind/. This method estimates the half-time of dissociation of a given MHC-peptide complex. HLA_BIND provides prediction for more than 40 different MHC class I types. It has been shown that profile based methods are correct in about 30% of the time, in the sense that one third of the predicted binders actually bind.

Yet another MHC-peptide predition method is SVMC, based on support vector machines to predict the binding of peptides to MHC class I molecules (Dönnes and Elofsson, BMC Bioinformatics 2002, 3:25). The prediction models for these MHC types are implemented in a public web service available at http://www.sbc.suae/svmhc/.

Brusic et al. reported the prediction of MHC class II-binding peptides using an evolutionary algorithm and artificial neural network (Bioinformatics, 1998; 14(2):121-30). Experimental binding data and expert knowledge of anchor positions and binding motifs were combined with an evolutionary algorithm (EA) and an artificial neural network (ANN): binding data extraction --> peptide alignment --> ANN training and classification. This method, termed PERUN, was implemented for the prediction of peptides that bind to HLA-DR4(B1*0401). Software and data are available from the authors upon request (vladimir@wehi.edu. au).

In a further aspect, a method of the invention comprises subjecting one or more polypeptide fragments predicted to bind to MHC and comprising one or more copies of a defined peptide motif sequence to a proteasome/immunoproteasome filter. Proteasomes are the main proteases responsible for cytosolic protein degradation and the production of major histocompatibility complex class I ligands. Incorporation of the interferon gamma- -inducible subunits low molecular weight protein (LMP)-2, LMP-7, and multicatalytic endopeptidase complex--like (MECL)-1 leads to the formation of immunoproteasomes which have been associated with more efficient class I antigen processing. Evidence suggests that the cleavage specificities of proteasomes play an important role in presenting peptides to the class-I MHC molecules. Therefore, researchers tried to improve the MHC binding predictions with the proteasomal cleavage predictions. Toes et al. (J. Exp. Med. 2001 July 2; 194(1):1-12) implemented filters for the proteasomal and immunoproteasomal cleavages on the predicted MHC binders, thus simulating two important steps of the antigen-processing pathway. According to one embodiment of the invention, a biologically active peptide is identified by its presence in a polypeptide fragment that is not only a predicted MHC binder but also predicted to be generated after proteasomal cleavage.

A further criterion that was identified by the present inventors to be an indicator or predictor of a peptide motif being biological active is related to the likelihood that the motif is exposed at the outer surface of a naturally occurring peptide. According to the invention, the presence of a peptide motif at an exposed surface has an increased chance of being released from the polypeptide, *e.g.,* upon proteolytic cleavage, and is therefore regarded as a positive indicator of the peptide motif being biologically active.

The invention thus includes a method for identifying a biologically active peptide consisting of two to seven amino acid residues, comprising the steps of: (i) providing a database comprising a plurality of naturally occurring polypeptide sequences; (ii) defining at least one peptide motif that satisfies one of the following criteria: AP, PA, A(P)n A, P(A)n P, wherein n = 0-5; (iii) determining for the defined peptide motif its frequency of occurrence among the polypeptide sequences in the database and correlating the frequency with the biological activity of the peptide, wherein a frequency of at least one is indicative of the peptide having a biological activity, and (iv) furthermore comprising the process of determining in silico the likelihood that the at least one peptide motif is exposed at the outer surface of the naturally occurring polypeptide.

The process of step (iv) comprising the steps of a) selecting at least one polypeptide sequence comprising at least one copy of at least one defined peptide motif; b) determining in at least one selected polypeptide the presence of one or more polypeptide regions having an increased likelihood of being exposed at the outer surface of the selected polypeptide; and c) selecting at least one defined peptide motif whose amino acid sequence is present in at least one of the exposed polypeptide regions, wherein exposure at the outer surface is a positive indicator of the peptide motif having biological activity.

The chance or likelihood that a polypeptide region is exposed (in aqueous environment) at the outer surface of the polypeptide can be determined using a hydrophilicity and/or hydrophobicity plots. These plots are designed to display or predict on the one hand hydrophobic membrane-spanning segments and on the other hand regions that are likely exposed on the surface of proteins (hydrophilic domains).

A hydrophilicity plot is a quantitative analysis of the degree of hydrophobicity or hydrophilicity of amino acids of a protein. It is used to characterize or identify possible structure or domains of a protein. The plot has amino acid sequence of a protein on its x-axis, and degree of hydrophobicity and hydrophilicity on its y-axis. There are a number of methods to measure the degree of interaction of polar solvents such as water with specific amino acids. For instance, the Kyte-Doolittle scale indicates hydrophobic amino acids, while the Hopp-Woods scale measures hydrophilic residues.

Analyzing the shape of the plot gives information about partial structure of the protein. For instance, if a stretch of about 20 amino acids shows positive for hydrophobicity, then this indicates that these amino acids may be part of alpha-helix spanning across a lipid bilayer, which is composed of hydrophobic fatty acids. Conversely, amino acids with high hydrophilicity indicate that these residues are in contact with solvent, or water, and that they are therefore likely to reside on the outer surface of the protein.In a method of the invention, a region of high hydrophilicity and/or low hydrophobicity is indicative of an increased likelihood of being outer surface exposed and thus of being (e.g., proteolytically) released from the polypeptide.

To generate data for a plot, a polypeptide sequence can be simply scanned with a moving window of some size. At each position, the mean hydrophobic index of the amino acids within the window is calculated and that value plotted as the midpoint of the window. Two of the most commonly used hydrophobicity scales are incorporated into the Hydropathy program.

Kyte-Doolittle scale: Hydropathic regions achieve a positive value. Setting window size to five to seven is suggested to be a good value for finding putative surface-exposed regions, whereas a window size of 19-21 yields a plot in which transmembrane domains stand out sharply, with values of at least 1.6 at their centers.

Hopp-Woods scale: This scale was developed for predicting potential antigenic sites of globular proteins, which are likely to be rich in charged and polar residues. This scale is essentially a hydrophilic index, with apolar residues assigned negative values. The authors suggest that, using a window size of six, the region of maximal hydrophilicity is likely to be an antigenic site.

In a method of the invention, the step of determining the probability that a peptide motif is outer surface exposed may be performed in any combination and in any order with one or more of the other selection criteria. By way of example, a method of identifying a biologically active peptide consisting of two to seven amino acid residues complying with one of the defined motids comprises as a first step determining the frequency of occurrence within a single polypeptide. Next, the polypeptide is subjected to an enzyme cutter program to determine which fragments are likely to be generated *in vivo.* Third, predicted fragments containing at least one intact copy of the peptide motif are selected. Fourth, it is determined whether the selected polypeptide fragments are surface exposed. Fifth, the surface exposed fragments are presented to an MHC class I and/or class II prediction method, optionally in combination with an (immuno)proteasome filter, to identify those fragments that are likely to be generated in the antigen-processing pathway. Peptide motifs which are present in the polypeptide fragments that satisfy all the criteria have a very high chance of being biologically active.

All of the above criteria were identified as being predictors of a given peptide sequence being biologically active or not. By using one or more predictors, the invention thus provides a method of identifying a biologically active peptide.

Once a biologically active peptide is identified, it would of course be valuable to know what kind of biological activity or activities the peptide would have. As indicated above, the expression "biological activity" is to be interpreted very broadly and therefore provides no indication for what purpose the peptide motif could be used, e.g., in a particular therapeutic or prophylactic treatment. A further aspect of the invention therefore relates to predicting the biological funtion of a peptide that has been identified (using the selection criteria set out herein above) as being biologically active.

Provided is a method for predicting the biological function of a defined peptide sequence of two to seven amino acid residues, comprising the steps of (i) identifying a biologically active peptide according to a method disclosed herein above; (ii) providing a database comprising multiple polypeptides, each having at least one known biological function; (iii) analyzing the frequency of occurrence of the amino acid sequence of the biologically active peptide sequence among the polypeptides in the database; (iv) selecting from the database at least one polypeptide, but preferably multiple polypeptides, comprising at least one copy of the biologically active peptide sequence and (v) identifying at least one biological pathway wherein the selected polypeptide is involved, wherein the predicted function of the defined peptide comprises is to modulate at least one identified biological pathway.

Typically, the database comprising multiple polypeptides, each having at least one known biological function, will be the same database which is used to identify the biologically active peptide motif. The identification method merely involves determining whether or not a defined peptide motif is present in whatever naturally occurring polypeptide. In contrast, the prediction method uses the known biological function of the polypeptide comprising the peptide motif to predict in which biological pathway the peptide motif will be active upon its production as a "virtual" breakdown product of the polypeptide.

Preferably, it involves selecting one, preferably more than one, polypeptide comprising one, preferably multiple, copies of a peptide motif identified to be biologically active. According to the invention, the predicted function of the peptide motif is to modulate the pathway wherein the one or more than one polypeptide is involved.

Modulation of a biological pathway by the peptide motif can be transiently or sustained. It can be stimulatory or suppressive. Modulation can be exerted at the level of the identified polypeptide itself, and/ or upstream and/or downstream thereof. Modulation can be achieved by a direct, e.g., protein-protein or DNA-protein, interaction between the peptide motif and at least one member of the pathway. In addition or as an alternative, modulation can be indirect, e.g., through the modulation of cellular levels of proteins, metabolites, signaling molecules and the like.

As used herein, the term "biological pathway" refers to any molecular interaction network involved in a biological process, for example any process responsible for a cell's activity, either chemical activity or physiological activity. Such pathways can involve gene expression levels, protein activation levels, the concentration of small molecules, external conditions (e.g., available nutrients) and other relevant biological processes and state. For instance, the peptide motif is predicted to modulate a metabolic pathway or a regulatory pathway.

The prediction method of the invention comprises the step of identifying at least one biological pathway wherein the polypeptide comprising one ore multiple copies of the peptide motif is involved. There are various publicly available databases with biological pathways that can be used to determine in which pathway(s) a selected polypeptide is involved.

The KEGG pathway (http://www.genome.jplkegg/pathway.html) is a collection of manually drawn pathway maps representing the knowledge on a wide variety of molecular interaction and reaction networks. For example, Metabolism (carbohydrate, energy, lipid, nucleotide, amino acid, glycan, polyketides and non-ribosomal peptides, cofactor/vitamin, secondary metabolite, xenobiotics), Genetic Information Processing, Environmental Information Processing, Cellular Processes and Human Diseases.

Major publicly available biological pathway resources, including the Kyoto Encyclopedia of Genes and Genomes (KEGG) www.genome.jp/kegg/pathway.html), GenMAPP (www.genmapp.org/) and BioCarta (www.biocarta.com), provide a large collection of biological pathway diagrams. Thus far, these resources have been used primarily to associate microarray gene-expression data with pathway diagrams to create a comprehensive overview and interpretation of the expression profiles.

In certain embodiments, the prediction method of the invention comprises performing an Ingenuity Pathway Analysis (IPA) to identify a biological pathway comprising at least one polypeptide comprising one or more copies of a peptide motif. Ingenuity Pathways Analysis is a web-based software application that enables biologists and bioinformaticians to identify the biological mechanisms, pathways and functions most relevant to their experimental datasets or genes of interest. An IPA search based can be on genes, proteins, diseases, processes, functions, and identifiers. In certain embodiments, a method of the invention for prediction the function of a biologically active peptide comprises performing an IPA search using the peptide motif as input to elucidate one or more biological networks wherein a polypeptide or polypeptides comprising the motif is/are involved.

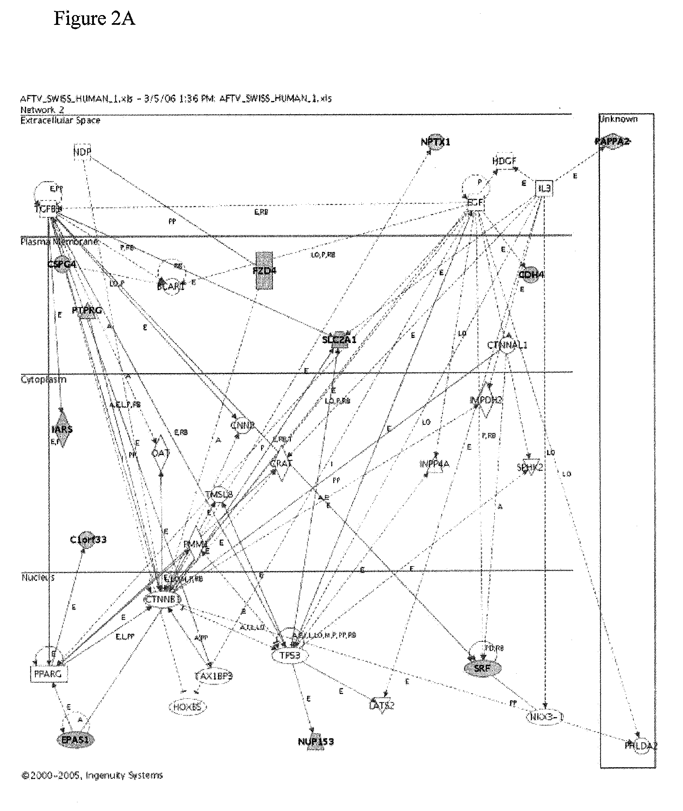

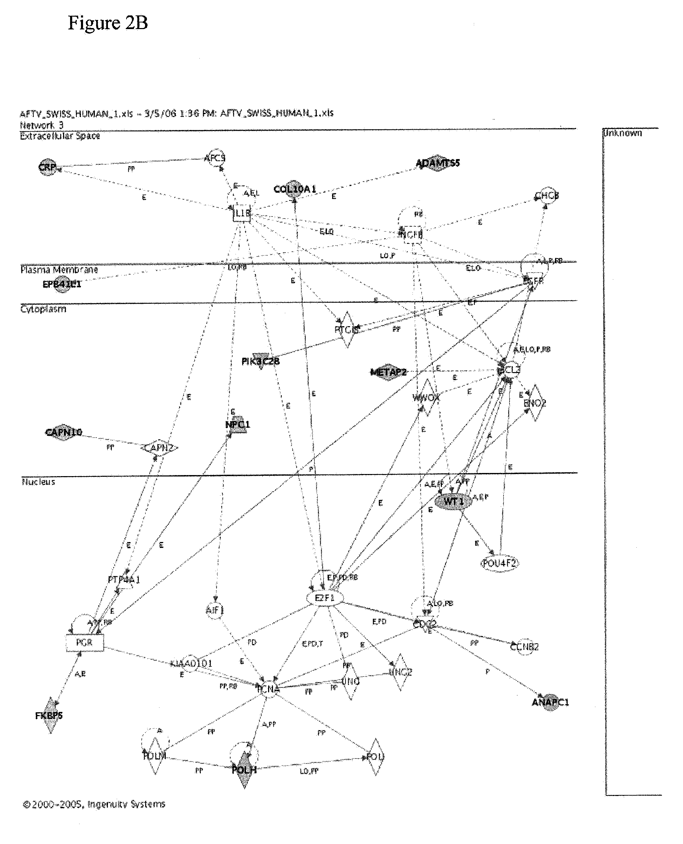

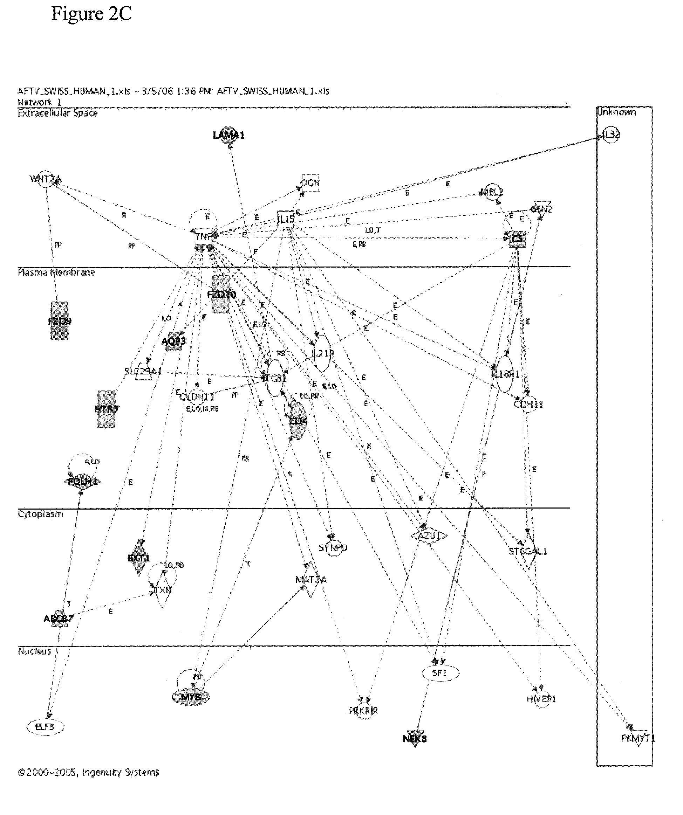

FIG. 2 illustrates some of the results that were obtained when peptide motif ATFV, present in the polypeptide C-reactiev protein (CRP) and identified as disclosed herein to be biologically active, was subjected to an Ingenuity Pathway Analysis. The ATFV sequence was found to be present in a number of naturally occurring human polypeptides (indicated in the figures in bold) playing a role in different biological pathways presented as different "networks" in FIGS. 2A, 2B and 2C. Within each network, it appears that polypeptides wherein the motif is represented act at various (sub)cellular sites, either extracellularly (e.g., LAMA1, NPTX1, COL10A1), at or close to the plasma membrane (e.g., FZD4, FZD9, FZD10, CD4, EPB41L1), in the cytoplasm (e.g., ABCB7, METAP2, IARS) or in the nucleus (e.g., MYB, NEK8, WT1, EPAS1).

According to the invention, peptide motif ATFV is predicted to function as a modulator of the networks shown in FIG. 2. Thus, one peptide can serve a modulatory role in more than one biological pathway or network.

Generally speaking, the more frequent a peptide motif is represented among the polypeptides making up the pathway or network, the more likely it is that the peptide motif has a modulatory role on or within the pathway or network. In particular, if a motif is represented in polypeptides that are involved within a pathway at different subcellular sites, this is a very strong predictor of the peptide motif being a regulator of the pathway.

Furthermore, it is possible to classify or rank the type of predicted biological function of a peptide motif to a certain class of biological activity based on the relative abundance of the motif among polypeptides involved in different types of networks. This can be used as further predictor of the biological activity of the peptide motif.

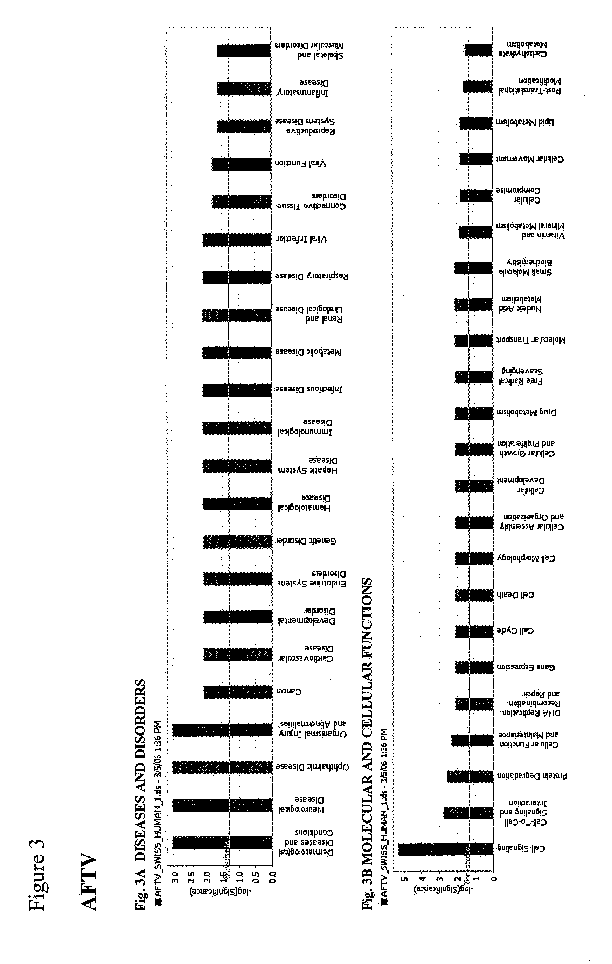

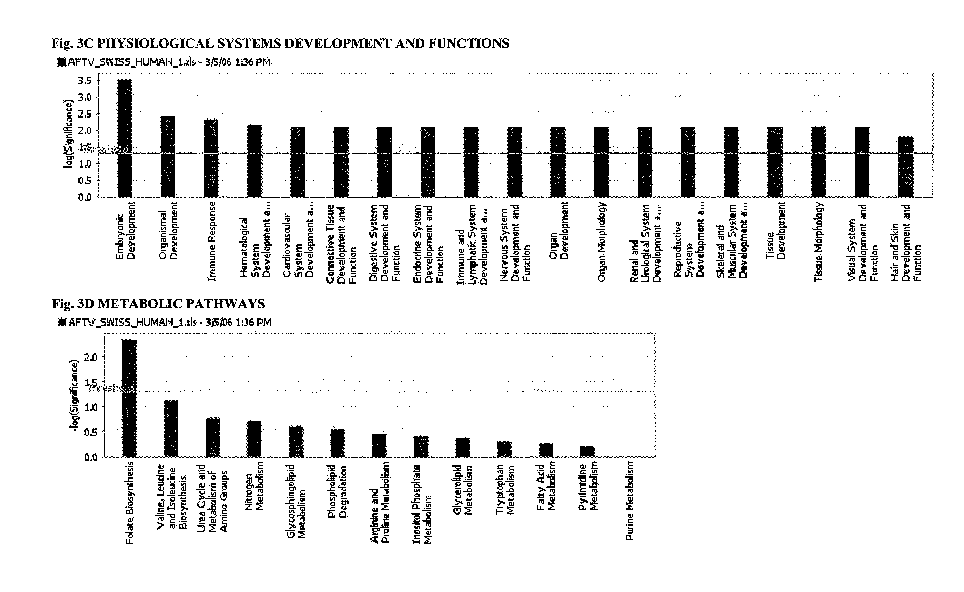

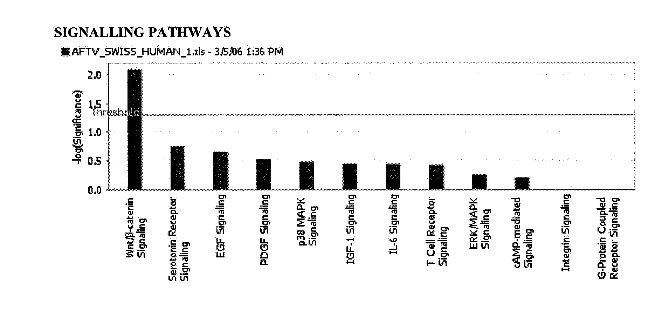

Continuing with the exemplary ATFV motif, this motif was found to be significantly overrepresented in cell signaling pathways in comparison with other pathways involved in molecular and cellular functions (see FIG. 3B). Further analysis of the different signaling pathways indicates that the ATFV peptide is in particular predicted to be a modulator of the Wnt/beta-catenin signaling pathway (results not shown). In addition, among pathways involved in diseases and disorders, the ATFV motif appears "enriched" in those underlying or related to dermatological diseases, neurological diseases, ophtalmic diseases, and organismal injuries and abnormalities (Fig 3A). With respect to physiological systems, development and functions, a modulatory function of embryonic development is predicted (FIG. 3C). Regarding metabolic pathways, ATFV is predicted to modulate folate biosynthesis (FIG. 3D).

Thus, the invention not only provides a method for identifying a biologically active peptide but also encompasses predicting its biological function, both in a normal and diseased state of an organism. Given the need in the art for improved methods of identifying hits and generating lead compounds for therapeutic purposes, the identification and prediction methods are clearly of great use in the field of drug discovery.

Accordingly, the invention also provides a method of conducting a drug discovery business comprising i) identifying one or more biologically active peptides using a peptide identification method according to the invention, ii) screening the peptide for the presence of descriptors indicative of a desirable therapeutic profile (e.g., ADME/T profile), iii) optionally modifying the peptide to improve its therapeutic profile; and iv) licensing, to a third party, the rights for further drug development of the peptide.

The method of conducting a drug discovery business may furthermore comprise predicting the biological function of the one or more biologically active peptides using a method of the invention and, optionally, correlating the predicted function with a disease or pathological condition.

In conducting therapeutic profiling of the peptide compound identified in step i), or further analogs thereof, for efficacy and toxicity in animals to generate lead compounds; iii) testing one or more lead compounds for efficacy and/or safety in human subjects, and iv) formulating and marketing a pharmaceutical preparation including one or more compounds of step iii), having an acceptable therapeutic profile. Such a business method can be further extended by including an additional step of establishing a distribution system for distributing the pharmaceutical preparation for sale, and may optionally include establishing a sales group for marketing the pharmaceutical preparation. Preferably, identifying lead peptide compounds includes screening hits for traits indicative of a desirable ADME/T profile and selecting peptide compounds having a higher probability of exhibiting a pharmaceutically desirable ADME/T profile.

The peptide motifs identified according to the invention may be prepared by methods known in the art (for example, see, U.S. Patent Application number 10/456,375). For example, by peptide synthesis methods known in the art, including, suitable N alpha protection (and side-chain protection if reactive side-chains are present). Protection of the α-amino group may utilize an acid-labile tertiary-butyloxycarbonyl group ("Boc"), benzyloxycarbonyl ("Z") group or substituted analogs or the base-labile 9-fluoremyl-methyloxycarbonyl ("Fmoc") group. The Z group can also be removed by catalytic hydrogenation, other suitable protecting groups include Nps, Bmv, Bpoc, Aloc, MSC, etc. A good overview of amino protecting groups is given in The peptides, Analysis, Synthesis, Biology, Vol. 3, E. Gross and J. Meienhofer, eds., (Academic Press, New York, 1981). Protection of carboxyl groups can take place by ester formation, for example, base-labile esters like methyl or ethyl, acid labile esters like tertiary butyl or, substituted, benzyl esters or hydrogenolytically. Protection of side-chain functions like those of lysine and glutamic or aspartic acid can take place using the aforementioned groups. Protection of thiol, and although not always required, of guanidino, alcohol and imidazole groups can take place using a variety of reagents such as those described in *The Peptides, Analysis, Synthesis, Biology,* or in Pure and Applied Chemistry, 59(3), 331-344 (1987). Activation of the carboxyl group of the suitably protected amino acids or peptides can take place by the azide, mixed anhydride, active ester, or carbodiimide method especially with the addition of catalytic and racemization-suppressing compounds like 1-N-N-hydroxybenzotriazole, N-hydroxysuccin-imide, 3-hydroxy-4-oxo-3,4-dihydro-1,2,3,-benzotriazine, N-hydroxy-5 norbornene-2,3-dicar-boxyimide. Also the anhydrides of phosphorus based acids can be used. See, e.g., *The Peptides, Analysis, Synthesis, Biology, supra* and Pure and Applied Chemistry, 59(3), 331-344 (1987).

It is also possible to prepare the peptides by the solid phase method of Merrifield. Different solid supports and different strategies are known see, e.g., Barany and Merrifield in The Peptides, Analysis, Synthesis, Biology, Vol. 2, E. Gross and J. Meienhofer, eds. (Acad. Press, New York, 1980); Kneib-Cordonier and Mullen, Int. J. Peptide Protein Res., 30, 705-739 (1987); and Fields and Noble, Int. J. Peptide Protein Res., 35, 161-214 (1990). The synthesis of peptide compounds in which a peptide bond is replaced by an isostere, can, in general, be performed using the previously described protecting groups and activation procedures. Procedures to synthesize the modified isosteres are described in the literature e.g., for the --CH2 --NH-- isostere and for the --CO--CH2isostere.

Removal of the protecting groups, and, in the case of solid phase peptide synthesis, the cleavage from the solid support may be performed by means known in the art *(see, e.g.,* volumes 3, 5 and 9 of the series on *The Peptides Analysis, Synthesis, Biology, supra).*

Another possibility is the application of enzymes in synthesis of such compounds; for reviews see, e.g., H. D. Jakubke in The Peptides, Analysis, Synthesis, Biology, Vol. 9, S. Udenfriend and J. Meienhofer, eds. (Acad. Press, New York, 1987). For example, by modifications such as glycosylation, phosphorylation and other modifications known in the art.

Peptides according to the invention may also be made according to recombinant DNA methods. Such methods involve the preparation of the desired peptide by means of expressing recombinant polynucleotide sequence which codes for one or more of the oligopeptides in a suitable host cell. Generally the process involves introducing into a cloning and/or expression vehicle (e.g., a plasmid, phage DNA, or other DNA sequence able to replicate in a host cell) a DNA sequence coding for the particular oligopeptide or oligopeptides, introducing the cloning and/or expression vehicle into a suitable eucaryotic or procaryotic host cell, and culturing the host cell thus transformed. When a eucaryotic host cell is used, the compound may include a glycoprotein portion.

In one aspect, sophisticated computer and statistical techniques is used to manage the data resulting from the methods of the invention. Preferably, the information generated is stored (or compiled) in electronic form, using a computerized database that allows information to be efficiently catalogued and retrieved. Such databases are comprised of records, typically one record per compound is generated, that includes information about the compound, stored as descriptors *(see,* J.M. Berger, "A Note on Error Detection Codes for Asymmetric Channels," Information and Control, Vol. 4, pp. 68-73, 1961; B. Bose and T.R.N. Rao, "Theory of Unidirectional Error Correcting/Detecting Codes," IEEE Transactions on Computers, Vol. C-31, No. 6, pp. 521-530, June 1982; R.W. Hamming, "Error Detecting and Error Correcting Codes," Bell Systems Technology Journal, Vol. 29, pp. 147-160, April 1950; D.E. Knuth, "Efficient Balanced Codes," IEEE Transactions on Information Theory, Vol. IT-32, pp. 51-53, 1986; Tom Verhoeff, "Delay-insensitive Codes: An Overview," Distributed Computing, Vol 3, pp. 1-8, 1988; Ramon Carrasco-Velar et al. "Definition of a novel atomic index for QSAR: the refractotopological state," J Pharm Pharmaceut Sci (2004) 7(1):19-26 (available online at ualberta.ca/∼csps); and E. J. Martin, et al. "Measuring Diversity: Experimental Design of Combinatorial Libraries for Drug Discovery" J. Med. Chem. (1995), 38(9):1431-1436).

The invention therefore also provides a computer program (software) capable of performing at least part of the steps comprised in the method for identifying a biologically active peptide and/or at least part of the steps comprised in the method of predicting the function of a biologically active peptide. The software can be referred to as "Peptide identification" or "Peptide-I" software.

The computer program for example comprises a motif search algorithm to compare a peptide motif with at least one protein from a protein sequence database and collect, classify, analyze and/or arrange protein sequence data (see Example 1).

In addition, the software may comprise a protein query algorithm to scan submitted protein sequences for motif patterns and filter the results based on user-defined selection criteria, and to provide as output a list of motifs that meet the selection criteria. Suitable selection criteria are one or more selected from a) the presence of one or more predicted cleavage sites flanking the motif, and preferably the absence a predicted cleavage site within the motif; b) the presence of a motif in polypeptide fragment(s) which are predicted to bind to class I and/or class II MHC molecules; and c) the exposure of a motif at the outer surface of a naturally occurring polypeptide. Furthermore, a computer program according to the invention may comprise an algorithm to build a "virtual" protein interaction network using functional information from a protein sequence database. Example 2 describes a representative protein query algorithm.

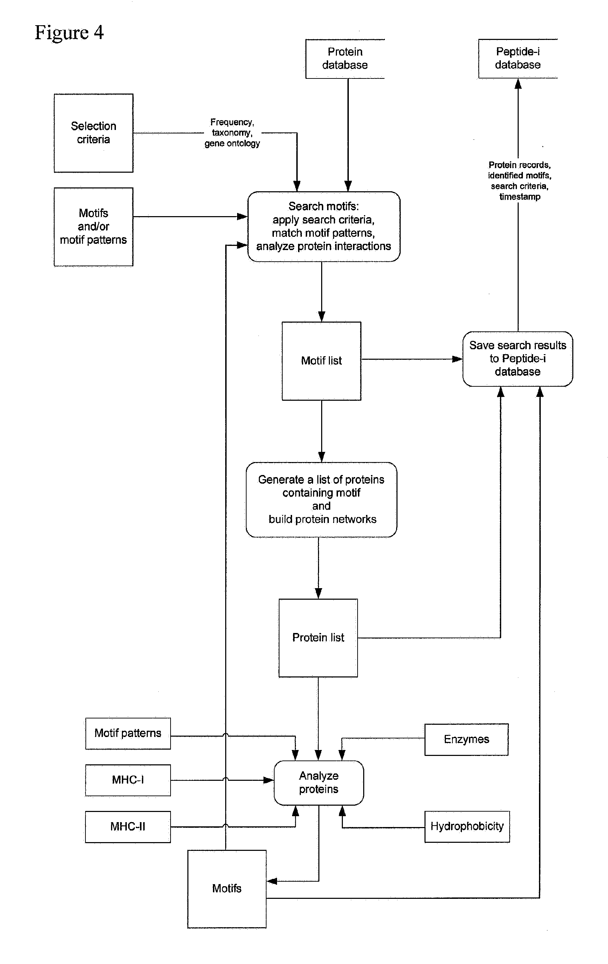

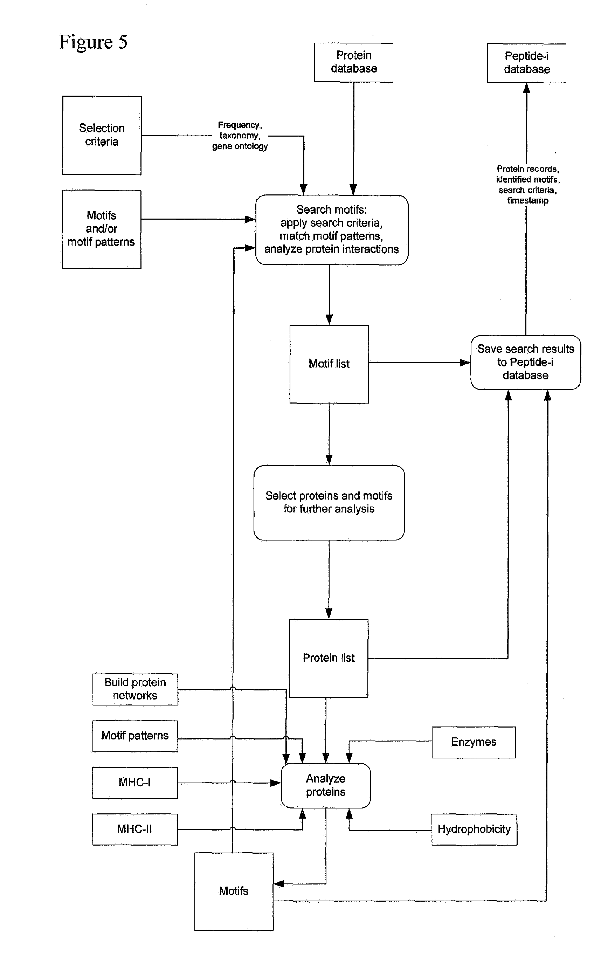

The motif search algorithm and the protein query algorithm are preferably integrated into a single computer program. The manner in which they are integrated can vary. FIGS. 4 and 5 show block diagrams illustrating exemplary computer programs suitable for use with the invention. The software may be written for Microsoft .NET platform in C#. Microsoft SQL server can be used as database.

Descriptors may be generated and/or entered into the database manually, that is by a user entering data through a user interface (e.g., keyboard, touchpad, etc.), or may be generated and/or entered electronically, for example, when a robotic system is used to generate results that are then converted to a descriptor and transferred to the database (often referred to as uploading). Such information may be stored in a discrete area of the record, e.g., the descriptor may refer to a sigle property or may describe multiple properties. As will be recognized, the information or descriptor, or a database of such information or descriptors, may be stored permanently or temporarily on various forms of storage media, including, but not limited to, compact disks, floppy disks, magnetic tapes, optical tapes, hard drives, computer system memory units, and the like.

The database may be stand-alone, or the records therein may be related to other databases (a relational database). Examples of other databases include publicly available databases, such as GenBank for peptides and neucleic acids (and associated databases maintained by the National Center for Biotechnology Information or NCBI), and the databases available through www.chemfinder.com or The Dialog Corporation (Cary, N.C.) for chemical compounds. The database may comprise wild-type ("normal") and /or mutated protein sequences. For example, polypeptides that are encoded by genes comprising one or more single nucleotide polymorphisms (SNPs) may be screened for the presence of a certain peptide motif.

A user will be able to search the database according to the information recorded (selecting records that have a particular value in a selected descriptor, for example, searching for all compounds that show the ability to up regulate NF2); accordingly, another aspect of the invention is a method of using a computer system to catalog and store information about various peptide motifs, their representation in naturally occurring polypeptides and the predicted biological function. The ability to store, retrieve and seach such information in computerized form allows those of ordinary skill in the art to select compounds for additional testing, including additional analysis of protein-protein interactions, physical characteristics of the compounds, toxicology testing in animal models, and/or clinical trials of pharmaceutical agents in humans. Moreover, in addition to single screening or searching, the database may be screened or searched using multiple individual protocols, the output thereof being searched to provide a report, either in electronic form or in the form of a printout, which will facilitate further analysis of selected compounds.

Also provided is a computer device comprising: a processor means, a memory means adapted for storing data relating to a plurality of protein sequences; means for inputting data relating to peptide motifs and a computer program stored in the computer memory adapted to screen the protein sequences for data relating to peptide motifs and outputting the screening results. The computer device for example comprises a computer program described above.

One embodiment of the invention comprises a computing environment; an input device, connected to the computing environment, to receive information from the user; an output device, connected to the computing environment, to provide information to the user; and a plurality of algorithms selectively executed based on at least a portion of the received information, wherein any one of these algorithms analyzes at least a portion of the received information and generates output information, and preferably wherein the output information is communicated via the output device. The computing environment preferably further comprises a communications network; a server connected to the network; and a client connected to the network, wherein the client is part of a client-server architecture and typically is an application that runs on a personal computer or workstation and relies on a server to perform some operations (see Nath, 1995, The Guide To SQL Server, 2nd ed., Addison-Wesley Publishing Co.).

The computing environment of the invention is advantageously implemented using any multipurpose computer system including those generally referred to as personal computers and mini-computers. Such a computer system will include means for processing input information such as at least one central processor, for example an Intel® processor (including Pentium® II3, Pentium® 4 or the like), or Motorola processor (for example, a PowerPC G4 microprocessor); a storage device, such as a hard disk, for storing information related to polypeptides and/or compounds; and means for receiving input information. Those of skill in the art recognize that computer technology is changing at a rapid rate; accordingly, the presently described components to illustrate the relationship of new, improved, versions of processors.

The processor, which comprises and/or accesses memory units of the computer system, is programmed to perform analyses of information related to the polypeptides and/or compounds. This programming may be permanent, as in the case where the processor is a dedicated PROM (programmable read-only memory) or EEPROM (electrically erasable programmable read-only memory), or it may be transient in which case the programming instructions are loaded from the storage device or from a floppy diskette or other transportable computer-readable media. The computing environment further preferably comprises a user interface such as a Unix/X-Window interface, a Microsoft Windows interface, or a Macintosh operating system interface.

Preferably, the computing environment further includes an optical disk for storing data, a printer for providing a hard copy of the data, and a monitor or video display unit to facilitate user input of information and to display both input and output information. The output information may be output from the processor within the computer system in print form using a printer; on a video display unit; or via a communications link or network to another processor or client application.

The invention is further explained with the aid of the following illustrative examples.

### EXAMPLES

### Example 1: Algorithms implemented in Peptide-I software

### Motif Search algorithm

The algorithm collects, classifies, analyzes and arranges protein sequence data in a particular output format based on a number of input criteria provided by the user.

The algorithm utilizes publicly available protein sequence data stored for quick access in a local relational database, such as Microsoft SQL Server or other database. The primary source of protein sequence data used by the algorithm is available from UniProtKB/Swiss-Prot (http://us.expasy.org/sprot/) - a curated protein sequence database.

First, the algorithm fetches protein sequences one by one from the local database and verifies the match of the protein information fields with the user-defined criteria. Such criteria could be: organism taxonomy, gene ontology, or any other description field related to the properties of the analyzed protein.

Second, the algorithm applies motif patterns defined by the user to identify matches in the sequence. Such motif patterns are denoted as regular expressions consisting of one or more amino acids specified by letters followed by typical regular expression characters, such as "*" to match zero or more occurrences of the acid, "+" to match one or more occurrences of the acid, "?" to match zero or one occurrence of the acid, "| " to match either of the acids, "[..]" to match any of the specified acids, "X" to denote all amino acids to simplify the structure of the motif pattern.

Third, to search for a motif pattern in a protein sequence, the algorithm utilizes regular expression matching, walks through all positions in the sequence, starting from the first position, and builds up a table of found matches together with the corresponding protein ids. The table is kept in memory for fast access in subsequent processing steps.

Fourth, the identified motif in the sequence is added to the table of found matches depending on the initial user choice for the number of occurrences of a motif in a sequence. For example, the user can specify that the motif should be stored if it occurs at least twice in the sequence.

Fifth, the algorithm analyzes protein binding data for all motifs stored in the table. The algorithm attempts to build a network of proteins using the protein-protein binding data from the UniProtKB/Swiss-Prot database or a similar protein database containing information on protein bindings. The network is constructed depending on the user choice for only first or first and second level of binding. With the first level of binding, a network is built up of proteins where motifs have been found. For example, if motif M1 is found in protein P1, and motif M2 is found in protein P2 that binds with protein P1, then both motifs are selected and belong to the P1-P2 network. With the second level of binding, the motifs are selected if proteins they are found in do not bind directly with each other, but do bind through other proteins. For example, if motif M1 is found in protein P1 that binds with protein P2, and motif M2 if found in protein P3 that binds with protein P2, than both motifs are selected and belong to the P1-P2-P3 network.

Sixth, the algorithm arranges the data to present the output in a convenient table format for easier analysis. Such a format depends on the user-defined search criteria, e.g., taxonomy, gene ontology, networks, etc., and contains input criteria vs. motif patterns, where each cell in the table contains the number of proteins in which the motif is found.

Seventh, the algorithm stores the results in a quickly searchable data structure, such as hash table, so the user is provided with fast access to any information on a protein or a group of proteins where a motif has been found.

### Query Proteins algorithm

The protein query algorithm scans submitted protein sequences for motif patterns and filters the results according to specified criteria. The output is a list of motifs that meet selection criteria and thus are likely to exit.

The algorithm can process amino sequences directly or utilize publicly available protein data stored for quick access in a local relational database, such as Microsoft SQL Server or other database. The primary source of protein data used by the algorithm is available from UniProtKB/Swiss-Prot (http://us.expasy.org/sprot/) - a curated protein sequence database.

The algorithm takes the amino sequences and applies the motif patters to identify motifs in sequences as described in 1.0004. For each motif, the positions where it's found in the sequence are kept. The same motif can have multiple occurrences in the sequence.

A number of tests, as described in the subsequent paragraphs, is then applied to the resulted list of motifs to determine which motifs are valid, i.e., are likely to exist.

First, short peptides are generated from longer protein sequences by the use of either chemical or enzymatic cleavage. The algorithm calculates the positions where the sequence is cut by the selected enzymes and/or chemicals. The entire list of motifs is then screened for positions in the sequence which are compared to the identified cut positions. The motif is considered valid only if it is not cut by any of the enzymes and/or chemicals.

Second, the algorithm uses a prediction method of MHC binding sites for Class-I molecules. Statistical/Mathematical expression based methods, including Quantitative matrix and Neural network based methods, are preferred. The method might be extended by allowing prediction of MHC binders for various alleles and proteasome cleavage site. The predicted MHC binders are then filtered based on prediction of proteasome cleavage sites in an antigenic sequence. For example, if a linear prediction model is used, the matrix data is calculated by summing up or multiplying the contribution of each amino acid. The peptides having scores more than a defined threshold score are assigned as binders. The entire list of motifs is then screened for positions in the sequence which are compared to the identified binders' positions. The motif is considered valid only if it falls entirely within the boundaries of the binder.

Third, the algorithm uses a prediction method of MHC binding sites for Class-II molecules. It works in a similar fashion as the algorithm in the preceding paragraph, but uses different alleles and different threshold scores to assign peptides as binders. The entire list of motifs is then screened for positions in the sequence which are compared to the identified binders' positions. The motif is considered valid only if it falls entirely within the boundaries of the binder.

Fourth, the algorithm considers the distribution of polar and apolar residues along a protein sequence using a hydrophobicity plot. The algorithm might incorporate any of the commonly used hydrophobicity scales, for example Kyte-Doolittle, or Hopp-Woods scale. Using the sliding window technique, the mean hydrophobic index of the amino acids within the window is calculated and that value is taken as the midpoint of the window. The entire list of motifs is then screened and compared to the hydrophobicity threshold.

Fifth, the algorithm analyzes protein binding data, for example using all motifs passed through the previous filtering steps. The algorithm attempts to build a network of proteins using the protein-protein binding data from the UniProtKB/Swiss-Prot database or a similar protein database containing information on protein bindings. The network is constructed depending on the user choice for only first or first and second level of binding. With the first level of binding, a network is built up of proteins where motifs have been found. For example, if motif M1 is found in protein P1, and motif M2 is found in protein P2 that binds with protein P1, then both motifs are selected and belong to the P1-P2 network. With the second level of binding, the motifs are selected if proteins they are found in do not bind directly with each other, but do bind through other proteins. For example, if motif M1 is found in protein P1 that binds with protein P2, and motif M2 if found in protein P3 that binds with protein P2, than both motifs are selected and belong to the P1-P2-P3 network.

## Claims

1. A method for identifying a biologically active peptide consisting of 2-7 amino acid residues, comprising:
providing a database comprising a plurality of naturally occurring polypeptide sequences; defining at least one peptide motif that satisfies one of the following criteria: AP, PA, A(P)ₙ A, P(A)n P,
wherein n = 0-5 and wherein A stands for an amino acid residue selected from a first subset of "A" amino acids and wherein P stands for an amino acid residue selected from a second subset of "P" amino acids, wherein said first and second subset are different from each other;
determining for said defined peptide motif its frequency of occurrence among the
polypeptide sequences in said database and correlating said frequency with the
biological activity of the peptide,
wherein a frequency of at least one is indicative of said peptide having a biological activity.

2. Method according to claim 1, wherein A is independently selected from the group consisting of amino acid residues Leu, Trp, Phe, Ile, Val, Pro, Ala, Met, Gly, Gln and Cys; and werein P is independently selected from the group consisting of amino acid residues Tyr, His, Thr, Lys, Ser, Arg, Glu, Gln, Cys, Asp, Asn, Pro, Ala, Val, Gly, Phe and Trp.

3. Method according to claim 1 or 2, comprising determining frequency of occurrence of said at least one motif among at least one single polypeptide sequence.

4. Method according to any one of claims 1 to 3, wherein the frequency is at least 5, preferably at least 10, more preferably at least 30.

5. Method according to any one of claims 1-4, wherein said biologic activity comprises a nuclear activity, preferably regulation of the transcription machinery.

6. Method according to any one of claims 1-5, wherein the motif is A(P)ₙ A, n = 1-5.

7. Method according to any one of claims 1-4, wherein said biologic activity comprises a cytosolic activity, preferably regulation of the signal transduction and/or the translation machinery.

8. Method according to any one of claims 1-4 or 7, wherein the motif is P(A)ₙ P, n = 1-5.

9. Method according to any one of the above claims, wherein said database is a database with human, viral, plant and/ or bacterial polypeptide sequences.

10. Method according to any one of the above claims, furthermore comprising the process of determining the likelihood that said peptide is generated *in vivo,* said process comprising:
a) selecting from said database at least one polypeptide sequence comprising at least one copy of at least one defined peptide motif;
b) determining in said at least one selected polypeptide the presence of one or more polypeptide fragments having an increased likelihood of being generated *in vivo;* and
c) selecting at least one defined peptide motif whose amino acid sequence is present in at least one of the polypeptide fragments.
wherein an increased likelihood is a further positive indicator of said peptide having a biological activity.

11. Method according to claim 10, wherein step b) comprises determining in *silico* the presence of a polypeptide fragment which is flanked by one or more predicted cleavage sites.

12. Method according to claim 11, wherein said cleavage site is an enzymatic and/or a chemical cleavage site, preferably a cleavage site recognized by an enzyme selected from the group consisting of the proteasome, immunoproteasome, Arg-C proteinase, Asp-N endopeptidase, caspase, Chymotrypsin, Clostripain (Clostridiopeptidase B, Enterokinase, Factor Xa, Glutamyl endopeptidase, GranzymeB, LysC, Pepsin (pH1.3), Pepsin (pH>2), Proline-endopeptidase, Proteinase K, Staphylococcal peptidase I, Thermolysin, Thrombin and Trypsin.

13. Method according to any one of claims 10-12, wherein step b) alternatively or additionally comprises determining in *silico* the presence of polypeptide fragment(s) which are likely to be generated in the antigen-processing pathway.

14. Method according to claim 13, wherein step b) comprises determining the presence of polypeptide fragment(s) which are predicted to bind to class I and/or class II MHC molecules.

15. Method according to claim 14, wherein said polypeptide fragment(s) are predicted binders to at least one class I MHC allele selected from the group consisting of HLA-alleles HLA-A*1101, HLA-A2.1, HLA-A*3302, HLA-B14, HLA-B*3701, HLA-B40, HLA-B*5103, HLA-B*51, HLA-B62, HLA-Cw*0301, H2-Db, H2-Kd, HLA-A2, HLA-A24, HLA-A68.1, HLA-B*2702, HLA-B*3801, HLA-B*4403, HLA-B*5201, HLA-B*5801, HLA-B7, HLA-Cw*0401, H2-Db, H2-Kk, HLA-A*0201, HLA-A3, HLA-A20 cattle, HLA-B*2705, HLA-B*3901,HLA-B*5101, HLA-B*5301, HLA-B60, HLA-B*0702, HLA-Cw*0602, H2-Ld, HLA-A*0205, HLA-A*3101, HLA-B*3501, HLA-B*3902, HLA-B*5102, HLA-B*5401, HLA-B61, HLA-B8,HLA-Cw*0702 and H2-Dd,H2-Kb.

16. Method according to any one of claims 13-15, wherein said polypeptide fragment(s) are predicted binders to at least one class II MHC allele selected from the group consisting of HLA-alleles HLA-DR1, HLA-DRB1*0101, HLA-DRB1*0102, HLA-DR3 HLA-DRB1*0301, HLA-DRB1*0305, HLA-DRB1*0306, HLA-DRB1*0307, HLA-DRB1*0308, HLA-DRB1*0309, HLA-DRB1*0311, HLA-DR4, HLA-DRB1*0401, HLA-DRB1*0402, HLA-DRB1*0404, HLA-DRB1*0405, HLA-DRB1*0408, HLA-DRB1*0410, HLA-DRB1*0423, HLA-DRB1*0426, HLA-DR7, HLA-DRB1*0701, HLA-DRB1*0703, HLA-DR8, HLA-DRB1*0801, HLA-DRB1*0802, HLA-DRB1*0804, HLA-DRB1*0806, HLA-DRB1*0813, HLA-DRB1*0817, HLA-DR11, HLA-DRB1*1101, HLA-DRB1*1102, HLA-DRB1*1104, HLA-DRB1*1106, HLA-DRB1*1107, HLA-DRB1*1114, HLA-DRB1*1120, HLA-DRB1*1121, HLA-DRB1*1128, HLA-DR13, HLA-DRB1*1301, HLA-DRB1*1302, HLA-DRB1*1304, HLA-DRB1*1305, HLA-DRB1*1307, HLA-DRB1*1311, HLA-DRB1*1321, HLA-DRB1*1322, HLA-DRB1*1323, HLA-DRB1*1327, HLA-DRB1*1328, HLA-DR2, HLA-DRB1*1501, HLA-DRB1*1502, HLA-DRB1*1506, HLA-DRB5*0101 and HLA-DRB5*0105.

17. Method according to any one of the above claims, furthermore comprising the process of determining in *silico* the likelihood that the at least one peptide motif is exposed at the outer surface of a naturally occurring polypeptide, said process comprising:
a) selecting at least one polypeptide sequence comprising at least one copy of said at least one defined peptide motif;
b) determining in said at least one selected polypeptide the presence of one or more polypeptide regions having an increased likelihood of being exposed at the outer surface of said selected polypeptide;
c) selecting at least one defined peptide motif whose amino acid sequence is present in at least one of the polypeptide regions; and
wherein an increased likelihood is a further positive indicator of said peptide having a biological activity.

18. Method according to claim 17, wherein step b) comprises subjecting the sequence of said at least one selected polypeptide to a hydrophilicity plot, wherein a region of high hydrophilicity is indicative of an increased likelihood of being outer surface exposed.

19. A method for predicting the biological function of a defined peptide sequence of 2-7 amino acid residues, comprising:
identifying a biologically active peptide according to any one of claims 1 to 17;
providing a database comprising multiple polypeptides, each having at least one known biological function;
analyzing the frequency of occurrence of the amino acid sequence of the biologically active peptide sequence among the polypeptides in said database;
selecting from the database at least one polypeptide comprising at least one copy of said biologically active peptide sequence; and
identifying in *silico* at least one biological pathway wherein said selected polypeptide is involved,
wherein the predicted function of said defined peptide comprises is to modulate said at least one identified biological pathway.

20. Method according to claim 19, wherein to modulate comprises to suppress or to activate said pathway.

21. A method of conducting a drug discovery business comprising i) identifying one or more biologically active peptides using a peptide identification method according to any one of claims 1 to 18, ii) screening the peptide for the presence of descriptors indicative of a desirable therapeutic profile, iii) optionally modifying the peptide to improve its therapeutic profile; and iv) licensing, to a third party, the rights for further drug development of the peptide.

22. Method according to claim 21, furthermore comprising the step of predicting the biological function of the biologically active peptide according to claim 19 or 20.

23. A computer program stored on a computer-readable medium for identifying a biologically active peptide, said computer program being capable of performing at least part of the steps comprised in the method according to any one of claims 1 to 18 and/or at least part of the steps comprised in the method of predicting the function of a biologically active peptide according to claim 19 or claim 20.

24. Computer program according to claim 23, comprising a motif search algorithm to compare a peptide motif with at least one protein from a protein sequence database and collect, classify, analyze and/or arrange protein sequence data.

25. Computer program according to claim 24, comprising a protein query algorithm to scan submitted protein sequences for motif patterns and filter the results based on user-defined selection criteria, and to provide as output a list of motifs that meet the selection criteria.

26. Computer program according to claim 25, wherein the selection criteria are one or more selected from:
a) the presence of one or more predicted cleavage sites flanking the motif, and preferably the absence a predicted cleavage site within the motif;
b) the presence of a motif in polypeptide fragment(s) which are predicted to bind to class I and/or class II MHC molecules; and
c) the exposure of a motif at the outer surface of a naturally occurring polypeptide.

27. Computer program according to claim 24, comprising an algorithm to build a protein interaction network using functional information from a protein sequence database.

28. A computer device comprising:
a processor means;
a memory means adapted for storing data relating to a plurality of protein sequences; means for inputting data relating to peptide motifs and a computer program stored in said computer memory adapted to screen said protein sequences for said data relating to peptide motifs and outputting the screening results.

29. Computer device according to claim 28, comprising a computer program according to claims 24-27.
